# EUROPEAN PATENT APPLICATION

(11) **EP 3 778 617 A1**
(43) Date of publication of application: **17.02.2021**
(21) Application number: 19776248.7
(22) Date of filing: 27.03.2019
(51) Int. Cl.: C07H 15/256, B01D 15/12, B01D 15/36, B01J 20/28, B01J 20/281, B01J 20/285, B01J 20/34, C07H 1/06, G01N 30/26, G01N 30/46, G01N 30/88, B01D 15/00

(54) **METHOD FOR SEPARATING STEVIOL GLYCOSIDE, METHOD FOR PRODUCING REBAUDIOSIDE A AND DEVICE FOR SEPARATING STEVIOL GLYCOSIDE**

(30) Priority: 27.03.2018 JP 2018060212
(71) Applicant: Mitsubishi Chemical Aqua Solutions Co., Ltd., Tokyo 141-0032 (JP)
(72) Inventor: NISHIMURA Kouji, Tokyo 100-0003 (JP); WATANABE Takahiro, Tokyo 100-0003 (JP); ADACHI Tadashi, Tokyo 100-8251 (JP)
(74) Representative: Hoffmann Eitle
(86) International application number: PCT/JP2019/013291
(87) International publication number: WO 2019/189422

(57) **Abstract**

A method for separating steviol glycoside, including: a separating step 55 of performing a continuous liquid chromatography for continuously separating at least one type of steviol glycoside by allowing a liquid to be separated containing plural types of steviol glycosides to pass through a separating agent in which polyethylene imine is immobilized to a carrier.

## Description

### TECHNICAL FIELD

The present invention relates to a method for separating steviol glycoside and the like, and more specifically, relates to a method for separating steviol glycoside and the like in which at least one type of steviol glycoside is separated from a liquid to be separated containing a plurality of types of steviol glycosides.

### BACKGROUND ART

Stevia that is an asteracea stevia plant has sweetness, and a stevia extracted material that is extracted from the stevia contains a plurality of types of steviol glycosides as a sweetness component. Specifically, in the steviol glycoside, rebaudioside A (C₄₄H₇₀O₂₃) and stevioside (C₃₈H₆₀O₁₈) are contained as a main component, and other derivatives are contained. Among them, the stevioside has a degree of sweetness of 300 times sugar, but also has bitterness or astringency. In contrast, the rebaudioside A only has a degree of sweetness of 450 times sugar, and does not have bitterness or astringency. Further, the rebaudioside A is a low calorie content, and thus, is suitable, for example, as a sweetener.

In Patent Literature 1, a method for purifying rebaudioside A from a composition containing rebaudioside A and stevioside by a recycle chromatography, with a separating device including at least four filling columns filled with a resin having a molecular sieve action, in which a styrene-based gel-type strongly acidic cation exchange resin having a moisture content of 50 mass% to 57 mass% is used as the resin, is disclosed.

In addition, in Patent Literature 2, a method for producing pure rebaudioside A, in which a hydrophilic vinyl polymer is used as a filtration gel for gel filtration for isolating rebaudioside A of sweetness components contained in an asteracea plant of stevia rebaudiana bertoni by using a gel filtration method, is disclosed.

### CITATION LIST

### PATENT LITERATURE

Patent Literature 1: JP 2011-51909 A
Patent Literature 2: JP 6-192283 A

### SUMMARY OF THE INVENTION

### PROBLEM TO BE SOLVED BY THE INVENTION

As described above, the rebaudioside A, for example, is suitable as a sweetener, and is required to separate the plurality of types of steviol glycosides contained in the stevia extracted material, particularly, to extract the rebaudioside A.

However, in the method of the related art, it is difficult to efficiently separate the plurality of types of steviol glycosides, and for example, in order to obtain the rebaudioside A of a high purity, there is a problem that great effort and time are required for a separating treatment.

An object of the invention is to provide a method for separating steviol glycoside and the like in which it is possible to efficiently attain a high purity at the time of separating at least one type of steviol glycoside from a plurality of types of steviol glycosides.

### MEANS FOR SOLVING PROBLEM

The present inventors have conducted studies for increasing a separation efficiency of a liquid chromatography of steviol glycoside by using a novel separating agent in which polyethylene imine is supported on a carrier. As a result thereof, an industrial process that is capable of efficiently performing separation with a high purity has been found, and thus, the invention has been completed.

According to the invention, a method for separating steviol glycoside, including: a separating step of performing a continuous liquid chromatography for continuously separating at least one type of steviol glycoside by allowing a liquid to be separated containing a plurality of types of steviol glycosides to pass through a separating agent in which polyethylene imine is immobilized to a carrier, is provided.

Here, the carrier can be a macromolecular carrier. In this case, the macromolecular carrier is more preferable as a carrier with respect to polyethylene imine.

Further, the liquid to be separated is capable of containing rebaudioside A as the steviol glycoside, and the rebaudioside A can be separated from the liquid to be separated. In this case, it is possible to efficiently separate the rebaudioside A from other steviol glycosides.

In addition, the liquid to be separated is capable of further containing stevioside as the steviol glycoside, and each of the rebaudioside A and the stevioside can be separated from the liquid to be separated. In this case, it is possible to efficiently separate the rebaudioside A and the stevioside.

Then, the liquid to be separated is capable of containing lower alcohol having carbon atoms of lower than or equal to 4 as a solvent. In this case, a separation efficiency of the steviol glycoside is further improved.

The continuous liquid chromatography can be performed by using a simulated moving bed type device. In this case, it is possible to make a high purity and a recovery efficiency of the steviol glycoside compatible.

In addition, according to the invention, it is possible to provide a method for producing rebaudioside A, including: a separating step of performing a continuous liquid chromatography for continuously separating rebaudioside A by allowing a liquid to be separated containing a plurality of types of steviol glycosides containing the rebaudioside A to pass through a separating agent in which polyethylene imine is supported on a carrier.

Further, according to the invention, it is possible to provide a device for separating steviol glycoside, including: a plurality of filling portions filled with a separating agent in which polyethylene imine for separating a plurality of types of steviol glycosides contained in a liquid to be separated by a chromatography is supported on a carrier; a supply portion provided in each of the filling portions to supply each of the liquid to be separated and an eluent for extracting a separated liquid rich in at least one type of steviol glycoside in the liquid to be separated to the filling portion; and an extraction portion provided in each of the filling portions to extract the separated liquid from the filling portion.

### EFFECT OF THE INVENTION

According to the invention, it is possible to provide a method for separating steviol glycoside and the like in which it is possible to efficiently attain a high purity at the time of separating at least one type of steviol glycoside from a plurality of types of steviol glycosides.

### BRIEF DESCRIPTION OF DRAWINGS

Fig. 1 is a diagram illustrating a separating flow of steviol glycoside to which this embodiment is applied;
Fig. 2 is a diagram describing a simulated moving bed type chromatographic separation device to which this embodiment is applied;
Fig. 3 is a flowchart describing an operation of a chromatographic separation device in this embodiment;
Figs. 4(a) and 4(b) are diagrams illustrating a concentration distribution of each a P component and an R component in a filling portion;
Figs. 5(a) to 5(h) are diagrams illustrating a direction of a flow of a liquid to be separated or an eluent in a filling portion in a case where two steps of step 101 and step 102 in Fig. 3 (a supplying and extracting step and a circulating step) are repeated four times (the first cycle to the fourth cycle);
Fig. 6 is a diagram illustrating a recycle type chromatographic separation device as another example of the chromatographic separation device;
Fig. 7 is a diagram illustrating a chromatogram at the time of using the chromatographic separation device illustrated in Fig. 6;
Fig. 8 is a diagram illustrating a chromatogram of Example A1;
Fig. 9 is a diagram illustrating a chromatogram of Example A2;
Fig. 10 is a diagram illustrating a chromatogram of Example A3;
Fig. 11 is a diagram illustrating a chromatogram of Comparative Example A1;
Fig. 12 is a diagram illustrating a chromatogram of Comparative Example A2;
Fig. 13 is a diagram illustrating a chromatogram of Comparative Example A3; and
Fig. 14 is a diagram illustrating a chromatogram of Comparative Example B1.

### MODE(S) FOR CARRYING OUT THE INVENTION

Hereinafter, a mode for implementing the invention will be described in detail. Note that, the invention is not limited to the following embodiments, and can be implemented by being variously modified within the scope of the gist thereof. In addition, the drawings to be used are for describing this embodiment, and do not represent the actual size.

### <Description of Liquid to Be Separated, Eluent, and Separating Agent>

### (Liquid to Be Separated)

A liquid to be separated of this embodiment is a liquid that is a target for separating a plurality of components to be contained by using a chromatographic separation device described below, and is a liquid in which a plurality of components are dissolved in a solvent such as water or an organic solvent. Then, the plurality of components are broadly separated into two fractions by using a difference in an interaction of each of the components with respect to a separating agent. In a case where the plurality of components, for example, are two components of a P component and an R component, the components are separated, and thus, any or both of the P component and the R component can be selectively extracted as a useful component. Note that, in the following description, a component having a larger interaction with respect to the separating agent is the P component, a component having a smaller interaction with respect to the separating agent is the R component (in a case where the interaction with respect to the separating agent is R Component < P Component), and the case of separating the P component and the R component will be described. That is, in this case, in a case where the liquid to be separated passes through the separating agent, a passing velocity of the R component is faster than a passing velocity of the P component. As a result thereof, the R component is likely to proceed first and the P component is likely to remain behind in a liquid passing direction. That is, the P component and the R component are separated. Note that, hereinafter, a liquid after the components are separated, which is a liquid rich in any of the P component and the R component, may be referred to as a "separated liquid".

Note that, the separation is not limited to a case where the component to be contained is two components, the component to be contained may be three or more components. Then, the separation can also be applied to the case of separating one component from the components, the case of broadly separating the component into two fractions, or the like.

In this embodiment, the liquid to be separated contains a plurality of types of steviol glycosides. The steviol glycoside to be contained, for example, is rebaudioside A, rebaudioside B, rebaudioside C, rebaudioside D, rebaudioside E, rebaudioside F, rebaudioside G, rebaudioside H, rebaudioside I, rebaudioside J, rebaudioside K, rebaudioside L, rebaudioside M, rebaudioside N, rebaudioside O, stevioside, dulcoside A, dulcoside B, rubusoside, and steviolbioside. In this case, it can also be described that the liquid to be separated contains the rebaudioside A, the stevioside, and a derivative of the rebaudioside A or the stevioside. Among them, the steviol glycoside that is contained in a stevia extracted material contains the rebaudioside A and the stevioside as a main component, as described above.

### (Eluent)

In this embodiment, an eluent is a liquid that is used for developing a component in a filling layer filled with the separating agent and for adjusting the size of an interaction between the separating agent and the component. The interaction between the separating agent and the component is adjusted by an eluent concentration, and thus, it is possible to separate and elute each of the components.

### (Separating Agent)

The separating agent adsorbs the component in the liquid to be separated. In this embodiment, a separating agent in which polyethylene imine is supported on a carrier is used as the separating agent.

### [1] Carrier

The carrier that is used in this embodiment is not particularly limited insofar as the carrier is not soluble in water or alcohol. For example, a macromolecular carrier formed of a macromolecular material or an inorganic carrier formed of an inorganic material is exemplified.

Examples of the macromolecular material configuring the macromolecular carrier include vinyl-based synthetic macromolecules, for example, styrene-based synthetic macromolecules such as polystyrene (PS), (meth)acryl-based synthetic macromolecules such as polymethyl methacrylate (PMMA), acetal-based synthetic macromolecules such as polyvinyl chloride (PVC), polyvinylidene chloride (PVDC), polyethylene (PE), polypropylene (PP), polyacrylonitrile (PAN), polyvinyl alcohol (PVA), and polyvinyl butyral, such as polyvinyl acetate (PVAc), polyacryl amide (PAA), polyvinyl ether-based synthetic macromolecules, and the like; diene-based synthetic macromolecules, for example, polybutadiene (PBd), polyisoprene (PIP), and the like; condensed synthetic macromolecules, for example, polyamide-based synthetic macromolecules such as nylon 6 and nylon 66, polyester-based synthetic macromolecules such as polyethylene terephthalate (PET) and polylactone, and polyether-based synthetic macromolecules such as polycarbonate (PC) and polyoxymethylene (POM); curable synthetic macromolecules, for example, a polyurethane resin, an alkyd resin, a phenol resin, polyaniline (PANI), and the like; epoxy-based synthetic macromolecules; silicone-based synthetic macromolecules; sulfur-based synthetic macromolecules such as xylene-based synthetic macromolecules, furane-based synthetic macromolecules, terpene-based synthetic macromolecules, petroleum-based synthetic macromolecules, ketone-based synthetic macromolecules, and cyclic polythioethes, and the like.

Examples of the inorganic material configuring the inorganic carrier include activated carbon, silica gel, diatom earth, hydroxyapatite, allumina, titanium oxide, magnesia, polysiloxane, and the like.

Among them, in this embodiment, the macromolecular carrier can be preferably used, and a macromolecular carrier formed of (meth)acryl-based synthetic macromolecules can be particularly preferably used. Further, it is preferable that the macromolecular carrier is porous particles.

The porous particles that are used in this embodiment, porous particles formed of cross-linkable (meth)acryl-based synthetic macromolecules can be preferably used. Examples of the porous particles include porous particles having a cross-linkage structure, which are obtained by polymerizing monovinyl (meth)acrylate and cross-linkable (meth)acrylate. Note that, herein, the (meth)acryl-based synthetic macromolecules indicates that greater than or equal to 50 weight%, preferably greater than or equal to 80 weight% of a monomer that is a raw material configuring a monomer polymer is a (meth)acryl-based monomer. Accordingly, the porous particles that are used in this embodiment may be porous particles in which greater than or equal to 50 weight% of all constituent units of macromolecules is a constituent unit derived from an acryl-based monomer or a methacryl-based monomer, and may have other constituent units derived from a polymerizable vinyl monomer. That is, preferably greater than or equal to 50 weight%, more preferably greater than or equal to 70 weight%, even more preferably greater than or equal to 90 weight% of all of the constituent units of the macromolecules is configured of the constituent unit derived from a methacryl-based monomer, from the viewpoint of excellent hydrolysis resistance and of improving an estimated usable period of the separating agent to be obtained.

Examples of the constituent unit configuring the (meth)acryl-based synthetic macromolecules that are used in this embodiment include a constituent unit derived from a (meth)acryl-based monomer or a polymerizable vinyl monomer that is exemplified as a raw material in a method for producing porous particles described below. In addition, a preferred range of a type, a ratio, or the like is also considered to be identical to that of the (meth)acryl-based monomer or the polymerizable vinyl monomer described below.

It is preferable that the porous particles that are used in this embodiment have a reactive functional group for immobilizing polyethylene imine having a specific molecular weight with a covalent bond. Examples of the reactive functional group include a hydroxyl group, an amino group, a carboxyl group, a halogen group such as a chloromethyl group, an epoxy group, and the like, and an epoxy group is preferable from the viewpoint of the ease of the introduction of a functional group and reactivity.

The reactive functional group can be introduced by selecting a constituent unit derived from a monomber having a functional group that can be a reactive functional group, as the constituent unit derived from a (meth)acryl-based monomer or a polymerizable vinyl monomer described above. Accordingly, the porous particles that are used in this embodiment are configured of a constituent unit derived from a (meth)acryl-based monomer including an epoxy group-containing (meth)acryl-based monomer. The constituent unit derived from a monomer having a functional group that can be a reactive functional group or a ratio thereof is considered to be identical to that of the raw material in the method for producing porous particles.

### (1) Method for Producing Porous Particles

The porous particles that are used in this embodiment, typically, can be produced by dispersing a monomer phase containing a polymerizable monovinyl monomer, a polymerizable polyvinyl monomer, a porosifier, a polymeric initiator, and the like in a water phase containing a dispersion stabilizer and the like, and by performing a polymerization reaction by heating or the like. Accordingly, spherical porous particles having a cross-linkage structure can be obtained. At this time, greater than or equal to 50 weight% of the polymerizable monovinyl monomer and the polymerizable polyvinyl monomer can be a (meth)acryl-based monomer. The reactive functional group of the porous particles can be introduced by selecting a monomer having a functional group that can be a reactive group, as the polymerizable monovinyl monomer or the polymerizable polyvinyl monomer described above.

Further, specifically, such porous particles, for example, can be produced by using a method disclosed in JP 58-058026 B or JP 53-090911 A. That is, the porous particles can be produced by a suspension polymerization or an emulsion polymerization between a (meth)acryl-based monomer having reactive functional group imparting properties and a (meth)acryl-based monomer or the like.

(Meth)acrylic acid esters such as methyl (meth)acrylate, ethyl (meth)acrylate, hydroxyethyl (meth)acrylate, hydroxypropyl (meth)acrylate, butyl (meth)acrylate, 2-ethyl hexyl (meth)acrylate, cyclohexyl (meth)acrylate, and glycerin mono(meth)acrylate, (meth)acryl amides such as (meth)acryl amide, dimethyl (meth)acryl amide, and hydroxyethyl (meth)acryl amide, nitriles such as (meth)acrylonitrile, an epoxy group-containing monomer such as glycidyl (meth)acrylate, 4,5-epoxy butyl (meth)acrylate, and 9,10-epoxy stearyl (meth)acrylate, and the like can be exemplified as the (meth)acryl-based monomer to be selected as the polymerizable monovinyl monomer.

In addition, in the polymerizable polyvinyl monomer for imparting a cross-linkage structure to the porous particles, a method for allowing a polyfunctional (meth)acryl-based monomer having a plurality of functional groups that can be polymerized in a polymerization reaction in the molecules to coexist is generally used. Examples of such a polyfunctional (meth)acryl-based monomer include alkylene glycol di(meth)acrylates such as ethylene glycol di(meth)acrylate and polyethylene glycol di(meth)acrylate, a polyvalent (meth)acryl compound such as alkylene di(meth)acrylate, N,N'-alkylene bis(meth)acryl amides, glycerol di(meth)acrylate, and trimethylol propane tri(meth)acrylate, a polyvalent allyl compound such as triallyl isocyanurate, diallyl phthalate, and the like.

As a use amount of the polymerizable polyvinyl monomer such as a polyfunctional (meth)acryl-based monomer, the amount of polyfunctional monomer is preferably greater than or equal to 5 weight% and less than or equal to 95 weight%, and is more preferably greater than or equal to 10 weight% and less than or equal to 90 weight%, with respect to entire monomer.

In a case where the polymerizable polyvinyl monomer is less than 5 weight%, the growth of the pore structure becomes insufficient, and the strength of polymer particles to be obtained decreases. On the other hand, in a case where the amount of polyfunctional monomer increases to be greater than 95 weight%, it is difficult to advance the immobilization of the polyethylene imine, an introduction amount decreases, and an adsorption amount of steviol glycoside is likely to be insufficient.

As described above, a polymerizable vinyl monomer other than the mono(meth)acryl-based monomer may be contained as the raw material of the porous particles that are used in this embodiment. In general, the polymerizable vinyl monomer may be contained in the amount of less than or equal to 30 weight%, preferably less than or equal to 20 weight%, more preferably less than or equal to 10 weight% of the entire monomer that is the raw material.

Examples of the polymerizable monovinyl monomer other that the mono(meth)acryl-based monomer include unsaturated carboxylic acids such as an itaconic acid and a maleic acid; styrene such as styrene, methyl styrene, ethyl styrene, α-methyl styrene, chlorostyrene, and chloromethyl styrene, and an alkyl or halogen substitute thereof; vinyl esters such as vinyl acetate and vinyl propionate, and vinyl ethers such as methyl vinyl ether and ethyl vinyl ether; allyl alcohol, and ester or ethers thereof; other vinyl compounds such as (meth)acrylonitrile, vinyl sulfonate, p-styrene sulfonate, vinyl pyridine, and vinyl pyrrolidone; and the like.

Examples of the polymerizable polyvinyl monomer other than the polyfunctional (meth)acryl-based monomer include an aromatic polyvinyl compound such as divinyl benzene, divinyl naphthalene, and 2,4,6-trivinyl ethyl benzene; polyvinyl polycarboxylic acid ester such as divinyl adipate, diallyl malate, diallyl phthalate, and 1,3,5-benzene triallyl tricarboxylate, polyallyl polycarboxylic acid esters; polyol polyvinyl ether such as divinyl ether, (poly)ethylene glycol divinyl ether, and polyol polyallyl ethers such as trimethylol propane diallyl ether and pentaerythritol triallyl ether; other polyvinyl compounds such as butadiene and methylene bisacryl amide; and the like.

Examples of the polymerizable vinyl monomer having reactive functional group imparting properties include a polymerizable monomer having a reactive functional group that is capable of immobilizing the polyethylene imine having a specific molecular weight with a covalent bond. Alternatively, a polymerizable monomer having a functional group that can be reacted with a compound (a linker) having such a reactive functional group is exemplified. In this embodiment, any of the monomers can be used.

The reactive functional group that is capable of immobilizing the polyethylene imine having a specific molecular weight with a covalent bond may be selected in accordance with the type of functional group corresponding to a bonding site of the polyethylene imine. For example, an epoxy group, a carboxyl group, and the like are exemplified. Among them, an epoxy group is preferable from the viewpoint of reactivity.

Examples of such a polymerizable monomer having an epoxy group include allyl glycidyl ether, vinyl glycidyl ether, 4-epoxy-1-butene, and the like, in addition to the epoxy group-containing monomer such as glycidyl (meth)acrylate, described above as an example of the (meth)acryl-based monomer. Among them, glycidyl (meth)acrylate is preferable, and glycidyl methacrylate is particularly preferable.

### [2] Polyethylene Imine

It is preferable that in the separating agent of this embodiment, the polyethylene imine is immobilized to the porous particles described above with a covalent bond.

It is preferable that in the polyethylene imine that is used in this embodiment, a mass average molecular weight (hereinafter, also simply referred to as a "molecular weight") is greater than or equal to 200 and less than or equal to 100000. The molecular weight of the polyethylene imine is preferably greater than or equal to 300, is more preferably greater than or equal to 500, is preferably less than or equal to 100000, and is more preferably less than or equal to 10000. In the separating agent of this embodiment, it is estimated that separating properties is improved in order for the polyethylene imine that is a functional group to three-dimensionally mutually interact with the steviol glycoside that is a separating target, and thus, in a case where the molecular weight is less than 200, the degree of interaction with respect to the steviol glycoside that is the separating target becomes insufficient. In addition, in a case where the molecular weight is greater than 100000, a viscosity increases, and thus, it is necessary that the polyethylene imine is diluted with a large amount of solvent in an immobilization reaction, a reaction rate of the immobilization reaction decreases, and the introduction amount with respect to the separating agent decreases, and as a result thereof, the adsorption amount of the steviol glycoside decreases.

Note that, such a molecular weight is a representative value, and specifically, satisfies a molecular weight represented in industrial polyethylene imine (Product Name: EPOMIN) distributed by Nippon Shokubai Co., Ltd., reagent polyethylene imine distributed by various reagent companies, or the like.

### (1) Reactive Functional Group of Porous Particles

As a method for immobilizing the polyethylene imine described above to the porous particles of the (meth)acryl-based synthetic macromolecules having a cross-linkage structure with a covalent bond, in general, the following methods are used, but the method is not limited thereto.

In the immobilization, a method for incorporating the (meth)acryl-based monomer having reactive functional group imparting properties in the particles of the (meth)acryl-based synthetic macromolecules by copolymerization or the like and for directly reacting the reactive functional group with the polyethylene imine can be used. In addition, a method for performing bonding through a low-molecular or macromolecular compound having one or more functional groups that can be reacted with each of the functional group contained in the constituent of the (meth)acryl-based synthetic macromolecules and the polyethylene imine in the molecules (hereinafter, such a compound will be collectively referred to as a "spacer") can be used.

For example, as the former method, a method for directly reacting the functional group in the particles of the (meth)acryl-based synthetic macromolecules, which forms a covalent bond with an amino group such as an epoxy group and a carboxyl group, with the polyethylene imine to be immobilized can be exemplified.

In addition, examples of the latter method include a method in which amino acids (aminocarboxylic acids) are used as a spacer, and an amino group site is reacted with an epoxy group of the (meth)acryl-based synthetic macromolecules, and then, is reacted with an amino group of the polyethylene imine by a carboxyl group on the other terminal. In addition, a method in which a polyglycidyl compound such as (poly)ethylene glycol diglycidyl ether and polyol polyglycidyl ether is used as a spacer, a hydroxyl group or an amino group of the (meth)acryl-based synthetic macromolecules and one terminal of the polyglycidyl compound are bonded, and an epoxy group on the other terminal and the polyethylene imine are bonded, and the like are exemplified.

Note that, in consideration of reactivity with respect to the polyethylene imine or a relationship of a steric barrier with respect to the particles of the (meth)acryl-based synthetic macromolecules in the immobilization, it is preferable that the spacer has a linear structure. In the case of using a spacer having a branched structure, probably because the steric barrier increases and the immobilization reaction of the polyethyne imine is suppressed, the adsorption amount tends to decrease.

### (2) Immobilization Reaction of Polyethylene Imine

In the immobilization reaction of the polyethylene imine, for example, the polyethylene imine is directly supplied to the porous particles of the (meth)acryl-based synthetic macromolecules having an epoxy group or the like, or is supplied as an organic solvent solution or an aqueous solution, and the reaction is performed.

In the case of independently using the polyethylene imine, the viscosity is high, and there is a problem in equipment for industrial production, and thus, it is preferable that the polyethylene imine is supplied to the (meth)acryl-based porous particles having an epoxy group or the like, as the organic solvent solution or the aqueous solution. Further, in the case of using the (meth)acryl-based porous particles having an epoxy group, in the aqueous solution, the immobilization reaction is a competition reaction with a diol formation reaction due to water addition with respect to an epoxy group, and thus, it is particularly preferable that the polyethylene imine is supplied to the porous particles of the (meth)acryl-based synthetic macromolecules having an epoxy group or the like, as the organic solvent solution.

It is preferable that the organic solvent is capable of dissolving the polyethylene imine, butyl alcohols, propyl alcohols, alcohols such as ethyl alcohol and methyl alcohol, ethers such as ethylene glycol dimethyl ether, diethylene glycol dimethyl ether, diethyl ether, tetrahydrofurane (THF), and dioxane, amides such as dimethyl formamide and dimethyl acetoamide, and the like are exemplified as the organic solvent, and ethers such as ethylene glycol dimethyl ether, diethylene glycol dimethyl ether, diethyl ether, THF, and dioxane, which swell the porous particles of the (meth)acryl-based synthetic macromolecules having an epoxy group or the like, are more preferable.

It is preferable that the temperature of the immobilization reaction is approximately a normal temperature (25°C) to 100°C. In a case where the temperature increases, there is a concern of the decomposition of the (meth)acryl-based porous particles, whereas in a case where the temperature is low, it takes a long time to perform the reaction.

### (3) Aftertreatment

After the immobilization reaction is performed as described above, it is preferable that a reactive functional group remaining on the porous particles side is inactivated by an aftertreatment. The reactive functional group remaining without being inactivated is gradually reacted with an active group such as steviol glycoside or foreign substances in a stevia leaf-extracted material, and thus, an adsorption capacity of the separating agent may be decreased or separating selectivity may be degraded.

In such an aftertreatment, for example, in a case where an epoxy group is an example of the reactive functional group, a method for reacting the epoxy group with water to be converted into diol can be exemplified. Examples of a catalyst at this time include an aqueous inorganic acid solution of a phosphoric acid, a sulfuric acid, and the like, and an aqueous alkali solution of sodium hydroxide, potassium hydroxide, and the like, and it is particularly preferable to use an aqueous sulfuric acid solution. A treatment condition of the aqueous sulfuric acid solution, such as a concentration, a reaction temperature, and a reaction time, is not particularly limited, and in general, the treatment can be implemented for 0.1 hours to 24 hours in a condition of a concentration of 1% to 30% and a temperature of 10°C to 90°C. Further, as a preferred treatment condition, the treatment is implemented for 1 hour to 10 hours at a concentration of 3% to 20% and a temperature of 20°C to 80°C.

### <Description of Separating Flow of Steviol Glycoside>

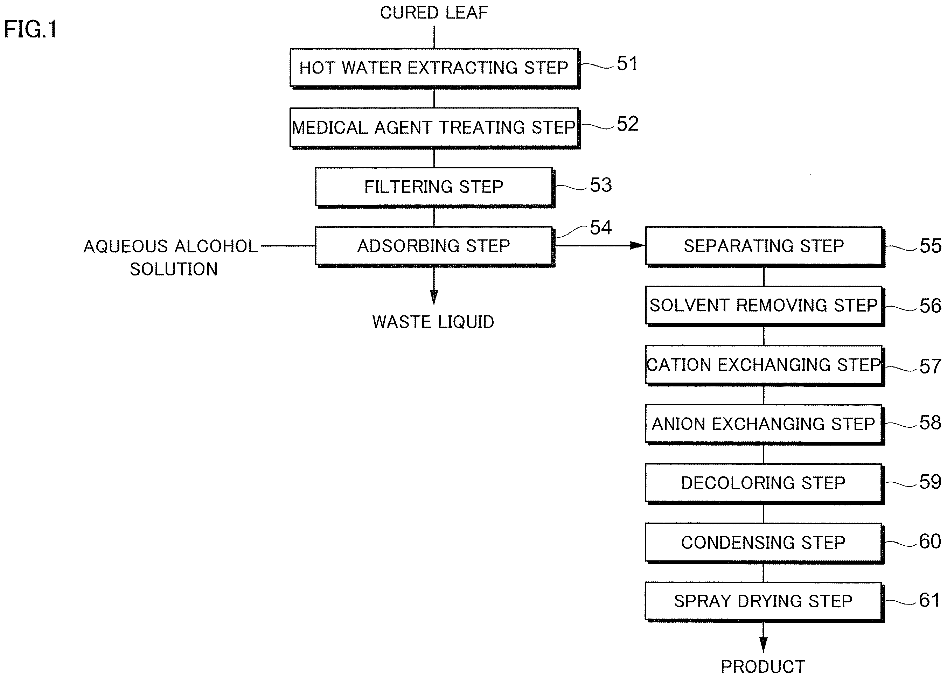

Fig. 1 is a diagram illustrating a separating flow of steviol glycoside to which this embodiment is applied. Note that, the separating flow of the steviol glycoside illustrated in Fig. 1 can also be attained by a method for separating steviol glycoside.

As illustrated, the separating flow of the steviol glycoside of this embodiment includes a hot water extracting step 51, a medical agent treating step 52, a filtering step 53, an adsorbing step 54, a separating step 55, a solvent removing step 56, a cation exchanging step 57, an anion exchanging step 58, a decoloring step 59, a condensing step 60, and a spray drying step 61.

In the hot water extracting step 51, a cured leaf obtained by drying a stevia leaf is used as a raw material, the cured leaf is put in hot water, and hot water extraction for extracting a component contained in the cured leaf is performed. The cured leaf that is the raw material, for example, is obtained by drying the stevia leaf at 20°C to 60°C for 1 hour to 24 hours. In addition, the leaf after drying may be further pulverized, and then, may be sued as the raw material. Note that, the extraction may be performed by using an organic solvent such as alcohol as a solvent, instead of water. In addition, it is possible to use a method such as supercritical fluid extraction for performing extraction by using a supercritical fluid, enzyme extraction for performing extraction by using an enzyme, microbe extraction for performing extraction by using a microbe, ultrasonic wave extraction for performing extraction by using an ultrasonic wave, and microwave extraction for performing extraction by using a microwave. An extracted liquid after the hot water extraction contains impurities in addition to a plurality of steviol glycosides.

In medical agent treating step 52, a medical agent is added to the extracted liquid subjected through the hot water extracting step 51, and a treatment for flocculating impurities is performed. Here, the charges on the surfaces of the impurities contained in the extracted liquid are neutralized, and flocculation is caused by a coagulation action for aggregating particles to be dispersed in the extracted liquid with a van der Waals' force (an intermolecular attractive force). That is, the medical agent is a flocculant, and for example, potassium alum (a double salt containing potassium ions, hydrated aluminum ions, and sulfuric acid ions), aluminum sulfate (Al₂(SO₄)₃), aluminum hydroxide (Al(OH)₃), aluminum oxide (Al₂O₃), diphosphorus pentoxide (P₂O₅), magnesium oxide (MgO), iron sulfate (FeSO₄), iron chloride (FeCl₃), and the like can be used.

In the medical agent treating step 52, the medical agent is added to the extracted liquid, is stirred for 5 minutes to 1 hour while adjusting the pH, for example, to be 8.5 to 9.0, and thus, the treatment is performed. The pH is adjusted, for example, by using calcium chloride (CaCl₂), calcium hydroxide (Ca(OH)₂), sodium hydroxide (NaOH), and the like.

In the filtering step 53, the impurities flocculated in the medical agent treating step 52 are removed by filtration. The filtration, for example, is performed by gravity filtration or centrifugal filtration using a filter.

In the adsorbing step 54, the steviol glycoside is adsorbed by using a synthetic adsorbent. In the adsorbing step 54, for example, a filtrate that is filtered through the filtering step 53 passes through one or a plurality of columns filled with the synthetic adsorbent, and thus, the treatment is performed. The synthetic adsorbent is configured of cross-linkage macromolecules having a porous structure, and for example, can be in the shape of a sphere. Examples of the synthetic adsorbent that can be used in the adsorbing step 54 include DIAION (Registered Trademark) HP20 manufactured by Mitsubishi Chemical Corporation, which is a styrene-divinyl benzene-based synthetic adsorbent, and SP700 manufactured by Mitsubishi Chemical Corporation, which is an aromatic-based synthetic adsorbent. Note that, the liquid that has passed through the synthetic adsorbent is a waste liquid.

The steviol glycoside that is adsorbed in the synthetic adsorbent is eluted by using an aqueous alcohol solution. Alcohol (an alcohol solvent) contained in the aqueous alcohol solution is not particularly limited insofar as the alcohol is freely mixed with water. For example, lower alcohol having carbon atoms of less than or equal to 4 is preferable, and lower alcohol having carbon atoms of less than or equal to 3 is more preferable, as the alcohol. Specifically, examples of the alcohol include methanol, ethanol, n-propanol, 2-propanol (isopropyl alcohol (IPA)), 1-butanol, 2-butanol, a mixture thereof, and the like. In addition, the alcohol, for example, has a concentration of 30 weight% to 95 weight% with respect to the entire aqueous alcohol solution.

It is possible to further remove the impurities by the adsorbing step 54. Then, the impurities are removed from the liquid to be separated subjected through the adsorbing step 54, and a plurality of steviol glycosides are contained in the liquid to be separated.

In the separating step 55, the separation of the plurality of steviol glycosides is performed. Specifically, in the separating step, a continuous liquid chromatography is performed in which the liquid to be separated passes through the separating agent, and thus, at least one type of steviol glycoside is continuously separated. The separating agent that is used at this time is the separating agent in which the polyethylene imine is supported on the carrier, described above. In the separating step 55, for example, the rebaudioside A is separated from other steviol glycosides. In addition, as described above, the steviol glycoside contained in the stevia extracted material contains the rebaudioside A and the stevioside as a main component, and thus, it is important to separate the main components from each other. Accordingly, it can also be described that in the separating step 55, the rebaudioside A and the stevioside are separated. A device for separating steviol glycoside that is used in the separating step 55 will be described below in detail.

In the solvent removing step 56, the alcohol solvent contained in the separated liquid subjected through the separating step 55 is removed. Specifically, the separated liquid is distilled or evaporated under reduced pressure, and thus, the alcohol solvent is removed.

In the cation exchanging step 57, the separated liquid subjected through the solvent removing step 56 passes through a column filled with a cation exchanger. Accordingly, a cation component in the separated liquid subjected through the solvent removing step 56 and an ion exchange group in the cation exchanger are subjected to ion exchange, and thus, the cation component is adsorbed. A cation exchange resin can be used as the cation exchanger.

The cation exchange resin is broadly divided into a strongly acidic cation exchange resin and a weakly acidic cation exchange resin, and any of the cation exchange resins can be used. Here, in this embodiment, a strongly acidic cation exchange resin is used.

Examples of the strongly acidic cation exchange resin include an acidic ion exchange resin having a sulfone group. In addition, in the case of sorting the cation exchange resin that is used in this embodiment in accordance with the degree of cross-linkage or a porosity, examples of the strongly acidic cation exchange resin include a gel-type cation exchange resin, a porous cation exchange resin, a high porous cation exchange resin, and the like, and any of the cation exchange resins can be used. Specifically, DIAION SK1B manufactured by Mitsubishi Chemical Corporation, which is a gel-type strongly acidic cation exchange resin, and the like can be used as the strongly acidic cation exchange resin.

Examples of the weakly acidic cation exchange resin include a cation exchange resin having a carboxyl group (-COOH) as an exchange group. In addition, a cation such as a metal ion and an ammonium ion (NH₄⁺) may be used instead of a hydrogen ion (H⁺) that is a counter ion of a carboxyl group. Specifically, DIAION WK40L manufactured by Mitsubishi Chemical Corporation, and the like can be used as the weakly acidic cation exchange resin.

In the anion exchanging step 58, the separated liquid subjected through the cation exchanging step 57 passes through a column filled with an anion exchanger. Accordingly, an anion component in the separated liquid subjected through the cation exchanging step 57 and an ion exchange group in the anion exchanger are subjected to ion exchange, and thus, the anion component is adsorbed. An anion exchange resin can be used as the anion exchanger.

The anion exchange resin is broadly divided into a strongly basic anion exchange resin and a weakly basic anion exchange resin, and any of the anion exchange resins can be used. Here, in this embodiment, a strongly basic anion exchange resin is used.

Examples of the strongly basic anion exchange resin include an anion exchange resin having a quaternary ammonium group as an exchange group. Specifically, DIAION HPA25L or PA308 manufactured by Mitsubishi Chemical Corporation, and the like can be used as the strongly basic anion exchange resin.

Examples of the weakly basic anion exchange resin include an anion exchange resin having a primary ammonium group or a secondary ammonium group as an exchange group. Specifically, DIAION WA10 manufactured by Mitsubishi Chemical Corporation, and the like can be used as the weakly basic anion exchange resin.

In the decoloring step 59, a deionized liquid subjected through the cation exchanging step 57 and the anion exchanging step 58 is decolored. The decoloration, for example, is performed by allowing the deionized liquid to pass through activated carbon. Accordingly, a coloration component is adsorbed in the activated carbon, and thus, the decoloration is performed.

In the condensing step 60, the condensation is performed by heating a decolored liquid subjected through the decoloring step 59, and by evaporating the moisture.

In the spray drying step 61, a condensed liquid subjected through the condensing step 60 is subjected to spray drying by using a spray drier.

According to the steps described above, for example, the rebaudioside A can be selectively separated from the plurality of steviol glycosides, and can be a product such as a sweetener. In addition, a method for producing rebaudioside A can be considered a method for separating the rebaudioside A by the steps described above.

Note that, the hot water extracting step 51, the medical agent treating step 52, the filtering step 53, and the adsorbing step 54 can be attained as a preparing step in which the liquid to be separated containing a plurality of types of steviol glycosides is prepared. Note that, the step is not limited thereto, and for example, the case of preparing the liquid to be separated, for example, by a purchase, can also be considered as the preparing step.

### <Description of Device for Separating Steviol Glycoside>

Next, the device for separating steviol glycoside that is used in the separating step 55 will be described in detail.

In this embodiment, a simulated moving bed type chromatographic separation device described below is used as the device for separating steviol glycoside.

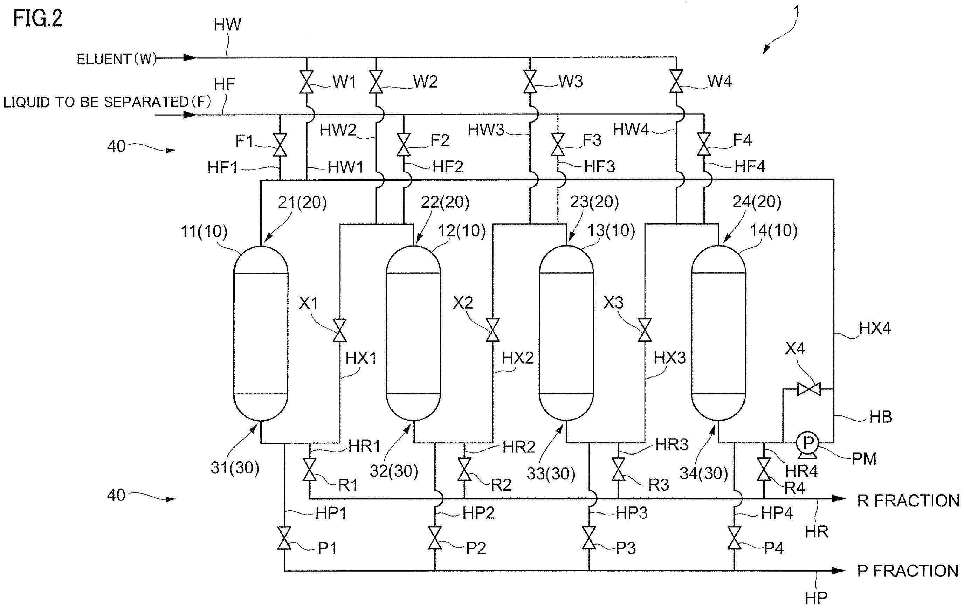

Fig. 2 is a diagram describing a simulated moving bed type chromatographic separation device 1 to which this embodiment is applied.

The chromatographic separation device 1 includes a filling portion 10 that separates a component, a supply portion 20 for supplying a liquid to be separated or an eluent, an extraction portion 30 for extracting a separated liquid, and a switching portion 40 for switching a flow path.

In this embodiment, four filling portions 10 are provided in one chromatographic separation device 1. In this embodiment, filling portions 11, 12, 13, and 14 (filling portions 11 to 14) are illustrated as the filling portion 10. Note that, hereinafter, in the case of not distinguishing the filling portions 11, 12, 13, and 14, the filling portions 11, 12, 13, and 14 may be simply referred to as the filling portion 10. The filling portion 10 is filled with the separating agent for separating a plurality of types of steviol glycosides contained in the liquid to be separated by a chromatography. The separating agent is the separating agent in which the polyethylene imine is supported on the carrier, described above. It is more preferable that a separation column filled with the separating agent is a packed column not having a void column in the upper portion. Note that, it is sufficient that the number of filling portions 10 is 2 in one chromatographic separation device 1. Here, it is more preferable that the number of filling portions 10 is greater than or equal to 3 from the viewpoint of a separation efficiency, and in a case where it is desirable to adjust the condition of the separating step, on the basis of the state of the liquid to be separated, and the like, it is even more preferable that the number of filling portions 10 is greater than or equal to 4. In addition, the number of filling portions 10 may be greater than or equal to 5 in one chromatographic separation device 1.

The filling portion 10, for example, is a column, and has a space to be filled with the separating agent inside. The filling portion 10, for example, is formed of a steel plate or the like, as a material, and a wetted portion can be subjected to rubber lining, but the filling portion is not limited thereto. For example, a resin or the like can also be used as the material of the filling portion 10. In addition, the shape of the filling portion 10 is not particularly limited, and in this embodiment, the filling portion 10, for example, has an approximately cylindrical shape, and has a columnar shape as a whole.

The supply portion 20 is provided in each of the filling portions 10 to supply each of the liquid to be separated and the eluent for extracting the separated liquid rich in at least one type of steviol glycoside in the liquid to be separated to the filling portion 10. The supply portion 20, for example, is a supply port provided in the upper portion of the filling portion 10. In this embodiment, supply portions 21, 22, 23, and 24 (supply portions 21 to 24) are illustrated as the supply portion 20. Note that, hereinafter, in the case of not distinguishing the supply portions 21, 22, 23, and 24 from each other, the supply portions 21, 22, 23, and 24 may be simply referred to as the supply portion 20.

The extraction portion 30 is provided in each of the filling portions 10 to extract the separated liquid from the filling portion 10. The extraction portion 30, for example, is a discharge port provided in the lower portion of the filling portion 10. In this embodiment, extraction portions 31, 32, 33, and 34 (extraction portions 31 to 34) are illustrated as the extraction portion 30. Note that, hereinafter, in the case of not distinguishing the extraction portions 31, 32, 33, and 34 from each other, the extraction portions 31, 32, 33, and 34 may be simply referred to as the extraction portion 30.

The switching portion 40, for example, is an on-off valve. Then, it is possible to switch the flow path of the liquid to be separated, the eluent, and the separated liquid by opening and closing the on-off valve. In this embodiment, the switching portion 40 includes an eluent on-off valves W1, W2, W3, and W4 (eluent on-off valves W1 to W4), liquid to be separated on-off valves F1, F2, F3, and F4 (liquid to be separated on-off valves F1 to F4), connection path on-off valves X1, X2, X3, and X4 (connection path on-off valves X1 to X4), R component on-off valves R1, R2, R3, and R4 (R component on-off valves R1 to R4), and P component on-off valves P1, P2, P3, and P4 (P component on-off valves P1 to P4).

The solubility of the steviol glycoside that is a solute in the liquid to be separated depends on a temperature, and thus, it is necessary to constantly maintain the temperature in the chromatographic separation device 1. Specifically, in a case where the steviol glycoside is eluted by using 30 weight% to 95 weight% of isopropyl alcohol (IPA) as a solvent, the temperature in the device is preferably 10°C to 50°C, is more preferably 20°C to 40°C, and is even more preferably 25°C to 35°C. In a case where the temperature in the device is lower than or equal to 10°C, the steviol glycoside is likely to be precipitated in the device, and thus, piping clogging or resin clotting is likely to occur, and as a result thereof, a drift is generated, and a liquid flow is likely to be degraded. In this case, there is a problem that the liquid to be separated is not capable of being continuously subjected to liquid passing in the device, and thus, it is difficult to attain industrialization. In addition, in a case where the temperature in the device is higher than or equal to 50°C, there is a concern that the steviol glycoside is modified by heating.

In order to constantly maintain the temperature in the device, the piping or the column may be covered with a thermal insulation material, or a thermal exchange device may be provided in a position where the temperature is likely to vary. In addition, in order to accelerate the dissolution of the liquid to be separated containing steviol glycoside, an ultrasonic wave may be applied.

In a case where the temperature in the chromatographic separation device 1 is 22°C to 35°C, and 30 weight% to 95 weight% of isopropyl alcohol (IPA) is used as a solvent, the concentration of the steviol glycoside in the liquid to be separated is preferably 5 g/L to 120 g/L, and is more preferably 10 g/L to 100 g/L. In a case where the concentration of the liquid to be separated is greater than 120 g/L, the steviol glycoside is likely to be precipitated in the device. In addition, in a case where the concentration of the liquid to be separated is less than or equal to 5 g/L, the separation efficiency decreases.

It is preferable that the chromatographic separation device 1 includes a pretreatment portion in order to reliably remove the impurities contained in the liquid to be separated (not illustrated). In the liquid to be separated containing a plurality of types of steviol glycosides, impurities such as a pigment component derived from a natural product or an organic acid are contained. In such impurities, a hydrophilic substance and a hydrophobic substance are respectively contained. Among them, adsorption based on an electrostatic interaction with respect to the hydrophilic substance having a negative charge is likely to occur between the hydrophilic substance and the polyethylene imine immobilized on the surface of the macromolecular carrier in the separating agent. In addition, adsorption based on a hydrogen bond or a hydrophobic interaction is likely to occur between the hydrophobic substance and (meth)acryl porous particles that are the macromolecular carrier. For this reason, an effective ion exchange group in the separating agent decreases, and thus, an ion exchange function of the separating agent decreases. In this case, even in a case where the separating agent is regenerated by sodium hydroxide, pure water, or the like, it is difficult to completely recover the function of the separating agent.

In addition, in a case where the impurities are contained in the liquid to be separated, a phenomenon occurs that the position or the height of the peaks of the P component, the R component, and other components is different for each of the filling portions 10, at the time of introducing the liquid to be separated and the eluent that are extracted from one filling portion 10 to the other filling portion 10. Then, in a case where positions of a plurality of peaks overlap each other, the purity of the component extracted from the extraction portion 30 decreases. In addition, in a case where the height of the peak decreases, a recovery rate of the component extracted from the extraction portion 30 decreases. Accordingly, in a case where the impurities contained in the liquid to be separated containing a plurality of types of steviol glycosides are not by reliably removed by the pretreatment, it is not possible to stably perform the continuous liquid chromatography.

The pretreatment portion, for example, is a precolumn, and includes a space to be filled with a separating agent inside. Then, the liquid to be separated containing the impurities such as a pigment component or an organic acid is subjected to liquid passing through the pretreatment portion to remove the impurities, and then, is supplied to the filling portions 11, 12, 13, and 14. Accordingly, it is possible to stably perform the continuous liquid chromatography. In consideration of an impurity removal rate or the ease of management, the separating agent filling the precolumn and the separating agent filling the filling portions 11, 12, 13, and 14 may be the same. A determination standard of whether or not the impurities can be removed is that a single-pipe column test is continuously performed, and the position or the height of the peaks of the P component, the R component, and the other components is not changed.

In the invention, the determination of whether or not the impurities are removed is performed by the single-pipe column test. In the single-pipe column test, the pretreated liquid to be separated passes through one single-pipe column two times consecutively, in the same condition as that of the continuous liquid chromatography that is performed after the pretreatment, and the concentration of the P component and the R component in each liquid subjected to the liquid passing with respect to a bed volume (BV) is measured.

In a change amount of BV of the peak top of the P component and the R component in the liquid that has passed through the single-pipe column in the second liquid passing with respect to BV of the peak top of the P component and the R component in the liquid that has passed through the single-pipe column in the first liquid passing, in general, in a case where the P component is less than or equal to 20% and the R component is less than or equal to 10%, preferably the P component is less than or equal to 10% and the R component is less than or equal to 5%, more preferably the P component is less than or equal to 5% and the R component is less than or equal to 3%, even more preferably the P component is less than or equal to 3% and the R component is less than or equal to 1%, it is possible to determine that the impurities are removed.

In a case where the separating agent filling the precolumn and the separating agent filling the filling portions 11, 12, 13, and 14 are the same, it is preferable that a condition for performing the pretreatment is the same as the condition of the chromatographic separation step. Specifically, in the case of eluting the steviol glycoside by using 30 weight% to 95 weight% of isopropyl alcohol (IPA) as a solvent, the temperature in the device is preferably 10°C to 50°C, is more preferably 20°C to 40°C, and is even more preferably 25°C to 35°C. In a case where the temperature in the device is lower than or equal to 10°C, the steviol glycoside is likely to be precipitated in the device, and thus, the piping clogging or the resin clotting is likely to occur, and as a result thereof, the drift is generated, and the liquid flow is likely to be degraded. In this case, there is a problem that the liquid to be separated is not capable of being continuously subjected to liquid passing in the device, and thus, it is difficult to attain industrialization. In addition, in a case where the temperature in the device is higher than or equal to 50°C, there is a concern that the steviol glycoside is modified by heating.

In order to constantly maintain the temperature in the device, the piping or the column may be covered with a thermal insulation material, or a thermal exchange device may be provided in a position where the temperature is likely to vary. In addition, in order to accelerate the dissolution of the liquid to be separated containing steviol glycoside, an ultrasonic wave may be applied.

In a case where the temperature in the single-pipe column is 22°C to 35°C, and 30 weight% to 95 weight% of isopropyl alcohol (IPA) is used as a solvent, the concentration of the steviol glycoside in the liquid to be separated is preferably 5 g/L to 120 g/L, and is more preferably 10 g/L to 100 g/L. In a case where the concentration of the liquid to be separated is greater than 120 g/L, the steviol glycoside is likely to be precipitated in the device. In addition, in a case where the concentration of the liquid to be separated is less than or equal to 5 g/L, the separation efficiency decreases.

In addition, the chromatographic separation device 1 includes piping HW for supplying the eluent from an eluent tank or the like, piping HW1 for supplying the eluent to the filling portion 11 from the piping HW, piping HW2 for supplying the eluent to the filling portion 12 from the piping HW, piping HW3 for supplying the eluent to the filling portion 13 from the piping HW, and piping HW4 for supplying the eluent to the filling portion 14 from the piping HW. In this case, the eluent on-off valve W1 to W4 are respectively provided in the pipings HW1 to HW4, and control the supply of the eluent with respect to the filling portions 11 to 14.

Further, the chromatographic separation device 1 includes piping HF for supplying the liquid to be separated from a liquid to be separated tank or the like, piping HF1 for supplying the liquid to be separated to the filling portion 11 from the piping HF, piping HF2 for supplying the liquid to be separated to the filling portion 12 from the piping HF, piping HF3 for supplying the liquid to be separated to the filling portion 13 from the piping HF, and piping HF4 for supplying the liquid to be separated to the filling portion 14 from the piping HF. In this case, the liquid to be separated on-off valve F1 to F4 are respectively provided in the pipings HF1 to HF4, and control the supply of the liquid to be separated with respect to the filling portions 11 to 14.

In addition, the chromatographic separation device 1 further includes piping HX1 for connecting the extraction portion 31 of the filling portion 11 and the supply portion 22 of the filling portion 12, piping HX2 for connecting the extraction portion 32 of the filling portion 12 and the supply portion 23 of the filling portion 13, piping HX3 for connecting the extraction portion 33 of the filling portion 13 and the supply portion 24 of the filling portion 14, piping HX4 for connecting the extraction portion 34 of the filling portion 14 and the supply portion 21 of the filling portion 11, as a connection path for connecting each of the filling portions 10. In this case, the connection path on-off valves X1 to X4 are respectively provided in the pipings HX1 to HX4, and control the circulation of the liquid to be separated between the filling portions 11 to 14.

Note that, a bypass path HB is provided at the point of the connection path on-off valve X4 in the piping HX4, and a pump PM is provided in the bypass path HB. Note that, the bypass path HB and the pump PM are provided in the piping HX4, but may be provided in any of the pipings HX1 to HX4, and may be provided in a plurality of positions in the pipings HX1 to HX4 (for example, all positions).

Then, the chromatographic separation device 1 includes piping HR for extracting the R fraction, piping HR1 for extracting the R fraction to the piping HR from the filling portion 11, piping HR2 for extracting the R fraction to the piping HR from the filling portion 12, piping HR3 for extracting the R fraction to the piping HR from the filling portion 13, and piping HR4 for extracting the R fraction to the piping HR from the filling portion 14. In this case, the R component on-off valves R1 to R4 are respectively provided in the pipings HR1 to HR4, and control the extraction of the separated liquid from the filling portions 11 to 14.

Then, the chromatographic separation device 1 includes piping HP for extracting the P fraction, piping HP1 for extracting the P fraction to the piping HP from the filling portion 11, piping HP2 for extracting the P fraction to the piping HP from the filling portion 12, piping HP3 for extracting the P fraction to the piping HP from the filling portion 13, and piping HP4 for extracting the P fraction to the piping HP from the filling portion 14. In this case, the P component on-off valves P1 to P4 are respectively provided in the pipings HP1 to HP4, and control the extraction of the separated liquid from the filling portions 11 to 14.

### <Description of Operation of Chromatographic Separation Device 1>

The chromatographic separation device 1 described above is operated as follows.

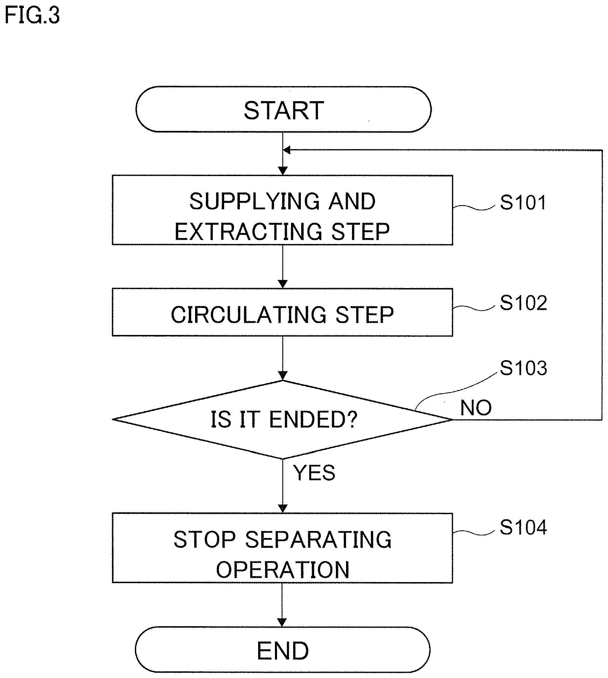

Fig. 3 is a flowchart describing the operation of the chromatographic separation device 1 in this embodiment.

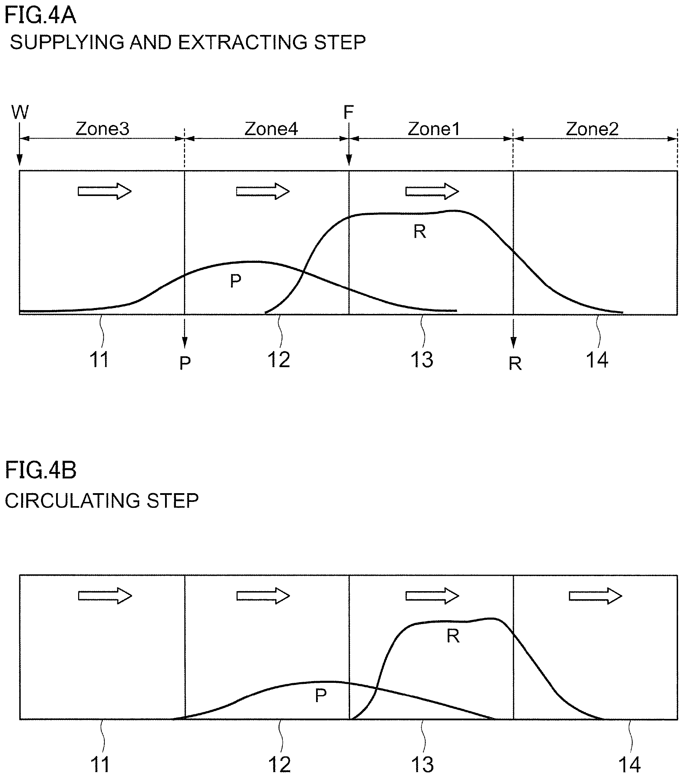

In addition Figs. 4(a) and 4(b) are diagrams illustrating a concentration distribution of each of the P component and the R component in the filling portions 11 to 14. Here, a horizontal direction represents a position in the filling portions 11 to 14. In each of the filling portions 11 to 14, in the drawing, the left side indicates the position of the upper portion in the filling portions 11 to 14 (on an upstream side), and in the drawing, the right side indicates the position of the lower portion in the filling portions 11 to 14 (on a downstream side). In addition, a vertical direction represents the concentration of the P component and the R component in each position. Further, a right arrow indicates the direction of the flow of the liquid to be separated and the eluent, and in this case, indicates that the liquid to be separated or the eluent flows into the filling portions 11 to 14 by a downward stream. Further, a case where the right arrow and the left arrow are not illustrated indicates that there is no flow in the filling portions 11 to 14. In addition, a lower arrow and an upper arrow represent a point for supplying the liquid to be separated or the eluent and a point for extracting the P fraction that is the separated liquid rich in the P component or the R fraction that is the separated liquid rich in the R component. In the drawing, the liquid to be separated is represented by "F", the eluent is represented by "W", the P component or the P fraction is represented by "P", and the R component or the R fraction is represented by "R".

Note that, in Fig. 4, the filling portion 13 may be referred to as an adsorption zone (Zone 1), the filling portion 14 may be referred to as a purification zone (Zone 2), the filling portion 11 may be referred to as a desorption zone (Zone 3), and the filling portion 12 may be referred to as a condensation zone (Zone 4). Then, in this embodiment, the separating operation is continuously performed while shifting the zones one by one.

In the filling portions 11 to 14, in a case where the liquid to be separated passes through the separating agent, as described above, the passing velocity of the R component is faster than the passing velocity of the P component. For this reason, for example, as illustrated in Fig. 4(a), the R component is likely to proceed first and the P component is likely to remain behind in the liquid passing direction. That is, in the filling portions 11 to 14, the P component and the R component are in a state of being separated.

Then, in the state of Fig. 4(a), each of the liquid to be separated and the eluent is supplied to different filling portions 10 of a plurality of filling portions 10 from each of the supply portions by a downward stream. In addition, the separated liquid rich in the P component and the separated liquid rich in the R component are respectively extracted from separate extraction portions (step 101: a supplying and extracting step).

In this case, the liquid to be separated on-off valve F3, the eluent on-off valve W1, the connection path on-off valves X1 and X2, the P component on-off valve P1, and the R component on-off valve R3 are opened, and the other on-off valves are closed. Accordingly, the liquid to be separated is supplied to the filling portion 13 from the supply portion 23, and the eluent is supplied to the filling portion 11 from the supply portion 21. In addition, the P fraction that is the separated liquid rich in the P component is extracted from the extraction portion 31, and the R fraction that is the separated liquid rich in the R component is extracted from the extraction portion 33.

That is, in the supplying and extracting step, the P component is eluted by the eluent that is supplied to the filling portion 11 from the supply portion 21, and the P fraction that is the separated liquid rich in the P component is extracted to the piping HP1 as a part of the supplied eluent. In addition, the residue of the eluent that is not extracted from the extraction portion 31 flows into the filling portion 12 from the piping HX1. Accordingly, the eluent is moved in a downward direction, and is circulated in the filling portion 12 and the filling portion 13. Then, in the filling portion 12 and the filling portion 13, the separation of the P component and the R component is advanced, and the concentration distribution of the P component and the R component is also moved to the downstream side. Then, the liquid to be separated is supplied to the filling portion 13, and the R fraction that is the separated liquid rich in the R component is extracted to the piping HR3 from the extraction portion 33 of the filling portion 13.

Here, a liquid amount that is extracted to the piping HP1 is a part of a liquid amount that is supplied from the supply portion 21. Accordingly, in order to control such a flow rate, it is necessary to attach a pump before the piping HP1 to perform the extraction at a constant flow rate or to adjust an extraction amount with an integrating flow meter.

Note that, in the supplying and extracting step, an operation of supplying the liquid to be separated from the supply portion 23 and of extracting the R fraction from the extraction portion 33, an operation of supplying the eluent from the supply portion 21 and of extracting the P fraction from the extraction portion 31, and an operation of supplying the eluent from the supply portion 21 and of extracting the R fraction from the extraction portion 33 may be respectively implemented by being divided.

Then, at a time point when step 101 is ended, the concentration distribution of the P component and the R component is as illustrated in Fig. 4(b).

Then, in the state of Fig. 4(b), the liquid to be separated and the eluent in the filling portion 10 are circulated between the filling portions 10 by a downward stream, and the separation of the plurality of components is advanced (step 102: a circulating step). Note that, at this time, the supply of the liquid to be separated and the eluent is not performed.

In this case, the connection path on-off valves X1, X2, and X3 are opened, and the other on-off valves are closed. Then, the pump PM is operated, and thus, the liquid to be separated and the eluent in the filling portion 10 are circulated between the filling portions 10 by a downward stream. That is, in this case, the connection path on-off valves X1, X2, and X3 are opened, and thus, all of the filling portions 10 are in a state of being connected by the pipings HX1, HX2, HX3, and HX4, and the bypass path HB, and a circulation path is formed. Then, the pump PM is operated, and the liquid to be separated or the eluent is moved in the circulation path. Here, the liquid to be separated and the eluent in the filling portion 10 are moved in the downward direction by one filling portion 10. In addition, at this time, the separation of the P component and the R component is advanced. As a result thereof, a concentration distribution having a shape shifted to the right side in the drawing by one is obtained with respect to the filling portion 10 from the state of Fig. 4(a). That is, a concentration distribution having a shape shifted by one is reproduced with respect to the filling portion 10 on the right side in the drawing. Accordingly, returning again to step 101, the same separating treatment can be repeated, and thus, the chromatographic separation can be continuously performed.

Then, whether or not to end the chromatographic separation is determined (step 103). A case where the chromatographic separation is ended, for example, is a case where water to be treated having an amount set in advance is treated. In addition, in a case where a pressure loss is greater than a size set in advance, the chromatographic separation may be ended, or when a separating operation time set in advance has elapsed, the chromatographic separation may be ended.

Then, in a case where the chromatographic separation is ended (Yes in step 103), the separating operation is stopped (step 104).

In contrast, in a case where the chromatographic separation is not ended (No in step 103), the process returns to step 101. That is, two steps of step 101 and step 102 described above are repeated.

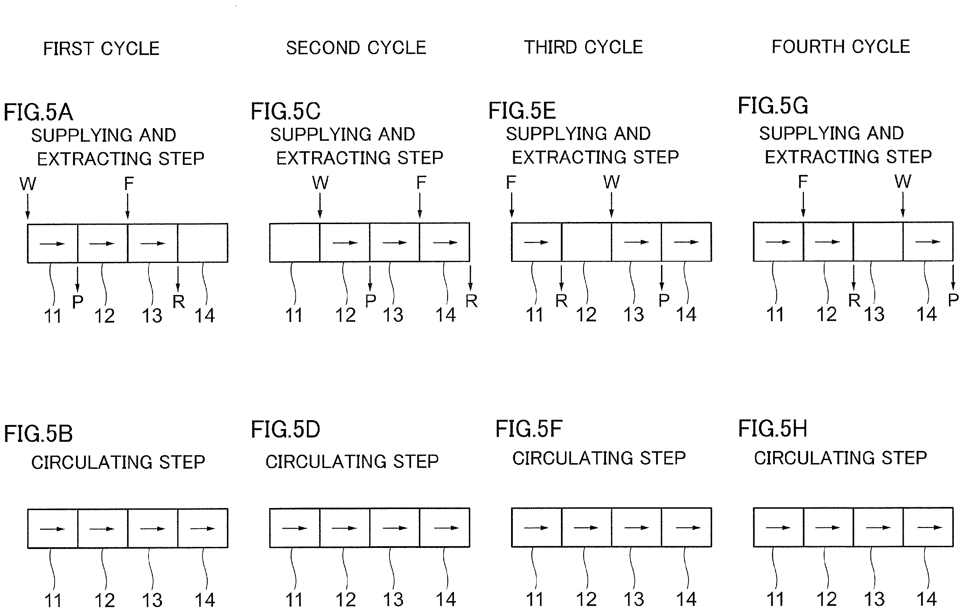

Figs. 5(a) to 5(h) are diagrams illustrating the direction of the liquid to be separated or the eluent in the filling portions 11 to 14 in a case where two steps of step 101 and step 102 in Fig. 3 (the supplying and extracting step and the circulating step) are repeated four times (the first cycle to the fourth cycle). Here, Figs. 5(a) and 5(b) are the first cycle, and Figs. 5(c) and 5(d) are the second cycle. In addition, Fig. 5(e) and (f) are the third cycle, and Figs. 5(g) and 5(h) are the fourth cycle. In addition, in Fig. 5, as with Fig. 3, the right arrow indicates the downward stream. Further, a case where the right arrow is not illustrated indicates there is no flow in the filling portions 11 to 14. Then, the lower arrow and the upper arrow represent the point for supplying the liquid to be separated or the eluent and the point for extracting the P fraction or the R fraction. In this case, the liquid to be separated is represented by "F", the eluent is represented by "W", the P component or the P fraction is represented by "P", and the R component or the R fraction is represented by "R".

In addition, Table 1 described below shows each of the on-off valves to be opened. Note that, on-off valves other than the on-off valves shown here are closed.

**[Table 1]**

| Cycle | Step | Supplied liquid | Extracted liquid | Opened on-off valve |
|---|---|---|---|---|
| 1 | Supplying and extracting step | Liquid to be separated Eluent | P | X1, X2, F3, W1, P1, R3 |
| | | | R | |
| | Circulating step | - | - | X1, X2, X3 |
| 2 | Supplying and extracting step | Liquid to be separated Eluent | P | X2, X3, F4, W2, P2, R4 |
| | | | R | |
| | Circulating step | - | - | X1, X2, X3 |
| 3 | Supplying and extracting step | Liquid to be separated Eluent | P | X3, F1, W3, P3, R1 |
| | | | R | |
| | Circulating step | - | - | X1, X2, X3 |
| 4 | Supplying and extracting step | Liquid to be separated Eluent | P | X1, F2, W4, P4, R2 |
| | | | R | |
| | Circulating step | - | - | X1, X2, X3 |

In the case of comparing Figs. 5(a) and 5(b), Figs. 5(c) and 5(d), Figs. 5(e) and 5(f), and Figs. 5(g) and 5(h), respectively, the position of the direction of the flow, the point for supplying the liquid to be separated or the eluent, and the point for extracting the P fraction or the R fraction are respectively shifted to the right side in the drawing (the downstream side) one by one with respect to the filling portion 10. In addition, with reference to Table 1, similarly, the position of the on-off valves to be opened are respectively shifted to the right side in the drawing (the downstream side) one by one with respect to the filling portion 10. Note that, in a case where one cycle is repeated four times, the state returns again to the original state. That is, the state returns to Fig. 5(a) after Fig. 5(h).

Here, the device for separating steviol glycoside that is used in this embodiment is not limited to the simulated moving bed type chromatographic separation device, but may be a device for performing a continuous liquid chromatography. Here, as with a batch system, the continuous liquid chromatography indicates not a treatment for repeatedly performing the supply of the liquid to be separated and the extraction of the separated component but a treatment for continuously extracting the separated component while continuously supplying the liquid to be separated.

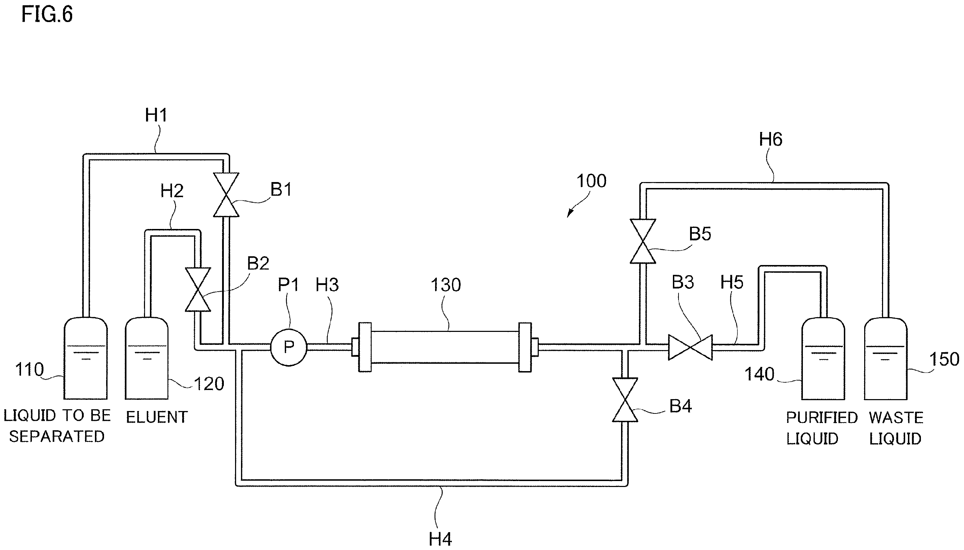

Fig. 6 is a diagram illustrating a recycle type chromatographic separation device as another example of the device for performing a continuous liquid chromatography.

A recycle type chromatographic separation device 100 that is illustrated includes a liquid to be separated tank 110 in which the liquid to be separated is accumulated, an eluent tank 120 in which the eluent is accumulated, a column 130 for separating the component contained in the liquid to be separated, purified liquid tank 140 in which a purified liquid after the component separation is accumulated, and a waste liquid tank 150 in which a waste liquid after the component separation is accumulated. In addition, the chromatographic separation device 100 further includes a pump P1, and on-off valves B1, B2, B3, B4, and B5.

In the chromatographic separation device 100, the on-off valve B1 is opened, and the on-off valve B2 is closed, and thus, the liquid to be separated is transferred to the column 130 from the liquid to be separated tank 110 through the piping H1 and the piping H3 by using the pump P1. Then, the on-off valve B1 is closed, and the on-off valve B2 is opened, and thus, the eluent is transferred to the column 130 from the eluent tank 120 in which the eluent is accumulated through the piping H2 and the piping H3. Then, the on-off valve B3 is opened, and the on-off valve B4 and the on-off valve B5 are closed, and thus, the purified liquid that passes through the column 130 and is discharged from the column 130 can be sent to the purified liquid tank 140 through the piping H5. Then, the on-off valve B3 and the on-off valve B4 are closed, and the on-off valve B5 is closed, and thus, the waste liquid that passes through the column 130 and is discharged from the column 130 can be sent to the waste liquid tank 150 through the piping H6.

On the other hand, the on-off valves B1, B2, and B3 and the on-off valve B5 are closed, and the on-off valve B4 is opened, and thus, the purified liquid that has passed through the column 130 can be returned again to the piping H3 through circulation piping H4. That is, it is possible to perform liquid passing through the column 130 a plurality of times by using the circulation piping H4.

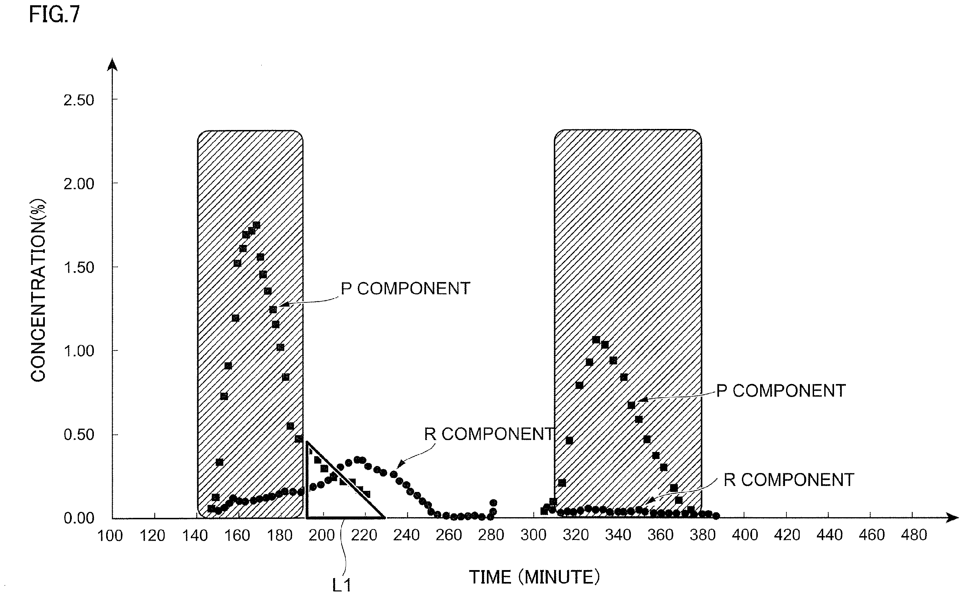

Fig. 7 is a diagram illustrating a chromatogram when the chromatographic separation device 100 illustrated in Fig. 6 is used, and the liquid to be separated passes through the column 130 five times to six times. In Fig. 7, a horizontal axis represents a time (minute), and a vertical axis represents the concentration of the component contained in the separated liquid.

In this case, the purified liquid discharged from the column 130 during 142 minutes to 190 minutes is returned again to the column 130 by using the circulation piping H4. Then, the purified liquid discharged from the column 130 during 190 minutes to 310 minutes is sent to the waste liquid tank 150. Further, the purified liquid discharged from the column 130 during 310 minutes to 380 minutes is sent to the purified liquid tank 140. In addition, the purified liquid discharged from the column 130 during 380 minutes to 480 minutes is further sent to the waste liquid tank 150. That is, a treatment in which a step of allowing a set of liquids to be separated to pass through the column 130 two times is set as one cycle is performed three times in total, and the separation of the P component and the R component is performed. Accordingly, it is possible to perform approximately the same separation by using a column 130 having a double length three times.

At this time, in the chromatogram, the P component at a point represented by L1 becomes a loss. In contrast, in the simulated moving bed type chromatographic separation device 1 described above, such a loss is less likely to occur, and the recovery efficiency is more excellent.

In addition, examples of the device for performing a continuous liquid chromatography further include a cyclic type chromatographic separation device (a cyclic chromatographic separation device or a cyclic chromatography device).

The cyclic type chromatographic separation device is a cross flow system in which a filler is moved such that a flow direction of an eluting agent becomes a cross flow, and thus, the liquid to be separated supply is continuously supplied and a plurality of components are continuously separated. In this system, a cyclic filling portion filled with the filler in a cyclic shape is rotatively moved such that the flow of the eluting agent and the liquid to be separated, flowing from the upper portion to the lower portion of the cyclic filling portion, becomes cross flow relatively. In the cyclic type chromatographic separation device, for example, a gap between an inner cylinder and an outer cylinder of a double cylindrical body that is rotated in a circumferential direction is filled with a filler to form the cyclic filling portion, the liquid to be separated and the eluting agent are continuously supplied to the cyclic filling portion from the upper portion, and the component separated from the lower portion is continuously extracted.

Note that, in the aspect described above, the opening and closing of the switching portion 40 that is an on-off valve may be manually controlled or may be automatically controlled. In addition, the case of manual control and the case of automatic control may be combined. In the case of automatic control, for example, a control unit such as a control board is provided, and the control unit performs the control of the switching portion 40 in cooperation with software and hardware resources. That is, a programmable logic controller (PLC) for control that is provided in the control unit reads a program for controlling the opening and closing of the switching portion 40, and executes the program, and thus, controls the opening and closing of the switching portion 40.

According to the simulated moving bed type chromatographic separation device 1 described above in detail, it is possible to improve the purity at the time of separating at least one type of steviol glycoside from the liquid to be separated containing a plurality of types of steviol glycosides. For example, in the case of separating the rebaudioside A and the stevioside, it is possible to attain a purity of greater than or equal to 95%. The purity of greater than or equal to 95% satisfies Generally Recognized As Safe (GRAS) that is a safety standard certificate given to food additives by the Food and Drug Administration (FDA) in the United States of America.

On the other hand, in the case of using the simulated moving bed type chromatographic separation device 1 described above in detail, the recovery efficiency is also excellent, and for example, greater than or equal to 98% of the rebaudioside A contained in the liquid to be separated can be recovered, and thus, the loss is small. That is, it is possible to make a high purity and the recovery efficiency compatible.

In addition, in the chromatographic separation device 1 described above, it is possible to perform decoloring with respect to the liquid to be separated. That is, in the aspect described above, the chromatographic separation device 1 is used in the separating step 55, but can also be used in the decoloring step 59. Accordingly, the chromatographic separation device 1 can be used in at least one of the separating step 55 and the decoloring step 59.

Note that, the treatment of the liquid to be separated, which is performed in the chromatographic separation device 1 described above, can also be attained by a method for producing rebaudioside A including the following steps of (i) and (ii). The step of (i) corresponds to the hot water extracting step 51, the medical agent treating step 52, the filtering step 53, and the adsorbing step 54, described above. In addition, the step of (ii) corresponds to the separating step 55 described above.
(i) A preparing step of preparing the liquid to be separated containing a plurality of types of steviol glycosides containing rebaudioside A
(ii) A separating step of performing the continuous liquid chromatography for continuously separating the rebaudioside A by allowing the liquid to be separated to pass through the separating agent in which the polyethylene imine is supported on the carrier

### EXAMPLES

Hereinafter, the invention will be described in more detail by using Examples, but the invention is not limited to Examples unless exceeding the gist thereof.

### [Single-Pipe Column Test]

Here, in order to check separating performance of a liquid to be separated containing a plurality of types of steviol glycosides containing rebaudioside A, single columns were respectively filled with the separating agent of this embodiment described above, and a separating agent of the related art, and a single-pipe column test was performed. Note that, in this case, a separating mode is a hydrophilic interaction liquid chromatography (HILIC: hydrophilic interaction chromatography) mode. In the separating mode, rebaudioside A and stevioside are eluted quickly in a case where the amount of water is large as a solvent, but are eluted slowly in a case where the amount of alcohol is large. Here, as described below, the rebaudioside A and the stevioside are eluted slowly by increasing the amount of alcohol, and separation is performed during the elution.

### (Example A1)

In Example A1, a separating agent produced as described below was used.

That is, 1400 parts by weight of diethylene glycol dimethyl ether and 600 parts by weight of polyethylene imine (manufactured by Wako Pure Chemical Industries, Ltd, a reagent, a molecular weight of 600) were added to 400 parts by weight of (meth)acryl-based porous particles that contain 70 parts by weight of glycidyl methacrylate and 30 parts by weight of ethylene glycol dimethacrylate and have a specific surface area of 37 m²/g, a pore diameter of 942 angstroms, and a pore volume of 0.99 mL/g, and were stirred to be in a suspension state. Such a suspension liquid was heated to 80°C, and was reacted for 6 hours. After cooling, the porous particles to which the polyethylene imine was immobilized were washed with water.

2000 parts by weight of an aqueous sulfuric acid solution of 10 weight% was added to the porous particles to which the polyethylene imine was immobilized, after being washed with water, and was stirred to be in a suspension state. Such a suspension liquid was heated to 50°C, and was retained for 5 hours, and thus, a diol formation reaction due to water addition with respect to an unreacted epoxy group was implemented.

After cooling, the porous particles were washed with water, and an ion exchange group was regenerated by an aqueous sodium hydroxide solution of 2 N, and thus, a separating agent was obtained.

In the separating agent that was obtained, an average particle diameter was 140 µm, a total exchange capacity was 2.99 milliequivalents/g, a specific surface area was 31 m²/g, a pore diameter was 944 angstroms, and a pore volume was 0.85 mL/g.

A column in which four columns having a diameter of 20 mmφ and a length of 250 mm are connected in series (a total length of 1000 mm) was filled with the separating agent. At this time, the amount of separating agent filling the column was 314 ml. Accordingly, in this case, 1 bed volume (BV) that is the amount of separating agent is 314 ml. Then, as a sample, Rebaudio JM-100 manufactured by MORITA KAGAKU KOGYO CO., LTD. was prepared and was set to an aqueous ethanol solution of 90%. In Rebaudio JM-100, rebaudioside A and stevioside are contained as a main component of steviol glycoside. In addition, in Rebaudio JM-100, other types of steviol glycosides or impurities are contained. Then, the aqueous ethanol solution of 90% passed through the column filled with the separating agent, and a single-pipe column test was performed. At this time, a flow velocity was 5.2 ml/minute. In this case, a space velocity SV is 1 h⁻¹. In addition, the aqueous ethanol solution of 90% was injected to the column at a room temperature of 28°C and 50 g/L in a total amount of 15.7 ml, and a chromatogram was measured with a UV detector wavelength of 210 nm.

### (Example A2)

In Example A2, the same separating agent as that of Example A1 was used.

In Example A2, the eluent was changed to aqueous isopropyl alcohol (IPA) solution of 89%, compared to Example A1. In addition, Rebaudio JM-100 that is a sample was injected to the column at a room temperature of 24°C and 10 g/L in a total amount of 15.6 ml. Then, otherwise a chromatogram was measured as with Example A1.

### (Example A3)

In Example A3, the same separating agent as that of Example A1 was used.

In Example A3, Rebaudio JM-100 that is a sample was injected to the column at a room temperature of 26°C and 100 g/L in a total amount of 15.6 ml, compared to Example A2. Then, otherwise a chromatogram was measured as with Example A2.

### (Comparative Example A1)

In Comparative Example A1, UBK530 (H type) that is a cation exchange resin for industrial chromatographic separation, manufactured by Mitsubishi Chemical Corporation, was used as a separating agent.

A single-pipe column having a diameter of 20 mmφ and a length of 1000 mm was filled with UBK530. At this time, the amount of UBK530 filling the column was 314 ml. Accordingly, in this case, 1 bed volume (BV) is 314 ml. Then, Rebaudio JM-100 that is a sample was set to an aqueous isopropyl alcohol (IPA) solution of 89%. Then, the aqueous isopropyl alcohol (IPA) solution of 89% passed through the column filled with the separating agent, and a single-pipe column test was performed. At this time, a flow velocity was 5.2 ml/minute. In this case, a space velocity (SV) is 1 h⁻¹. In addition, the aqueous isopropyl alcohol (IPA) solution of 89% was injected to the column at a room temperature of 25°C and 10 g/L in a total amount of 15.7 ml, and a chromatogram was measured with a UV detector wavelength of 210 nm.

### (Comparative Example A2)

In Comparative Example A2, a chromatogram was measured as with Comparative Example A1, except that the separating agent was changed to Sepabeads FP-DA13 (OH type) that is a styrene-based dimethyl amine type weakly basic anion exchange resin, manufactured by Mitsubishi Chemical Corporation, compared to Comparative Example A1.

### (Comparative Example A3)

In Comparative Example A3, a chromatogram was measured as with Comparative Example A1, except that the separating agent was changed to DIAION WA20 (OH type) that is a styrene-based polyamine type weakly basic anion exchange resin, manufactured by Mitsubishi Chemical Corporation, compared to Comparative Example A1.

### (Comparative Example A4)

In Comparative Example A4, a chromatogram was measured as with Comparative Example A1, except that the separating agent was changed to DIAION WA33L (OH type) that is a styrene-based dimethyl amine type weakly basic anion exchange resin, manufactured by Mitsubishi Chemical Corporation, compared to Comparative Example A1.

### [Separating Test Using Simulated Moving Bed Type Chromatographic Separation Device]

### (Example B1)

In Example B1, the simulated moving bed type chromatographic separation device 1 illustrated in Fig. 2 was used, and a separating test of the steviol glycoside was performed. At this time, the same separating agent as that of Example A1 was used.

An aqueous solution in which the concentration of a raw material was 10 mg/mg was prepared as a liquid to be separated. In the raw material, rebaudioside A and stevioside were contained as a main component, and a mass ratio thereof was 51:44.

In addition, in the raw material, other types of steviol glycosides or impurities are contained. In addition, an aqueous isopropyl alcohol (IPA) solution of 89% was used as an eluent. In addition, a space velocity SV when the device was operated was 1 h⁻¹. In addition, a load (a load amount) that is an injection amount of the liquid to be separated to the separating agent per 1 hour was 0.058 h⁻¹.

### (Comparative Example B1)

In Comparative Example B1, the same separating agent as that of Example B1 was used. Then, a single-pipe column having a diameter of 20 mmφ and a length of 1000 mm was filled with the separating agent. At this time, the amount of separating agent filling the column was 314 ml. The same liquid to be separated and eluent as those of Example B1 alternately passed through the single-pipe column, and a separating test of the steviol glycoside was performed. At this time, a flow velocity was 5.2 ml/minute. At this time, a space velocity SV was 1 h⁻¹. In addition, a load (a load amount) was 0.038 h⁻¹. Note that, hereinafter, in this method, the method for separating a component may be referred to as a "single-pipe column continuous treatment".

### [Result]

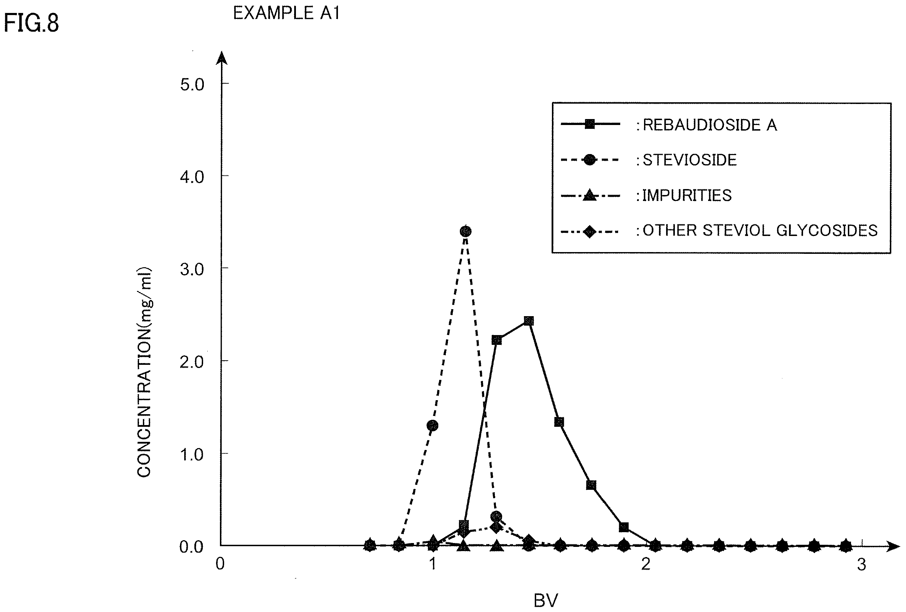

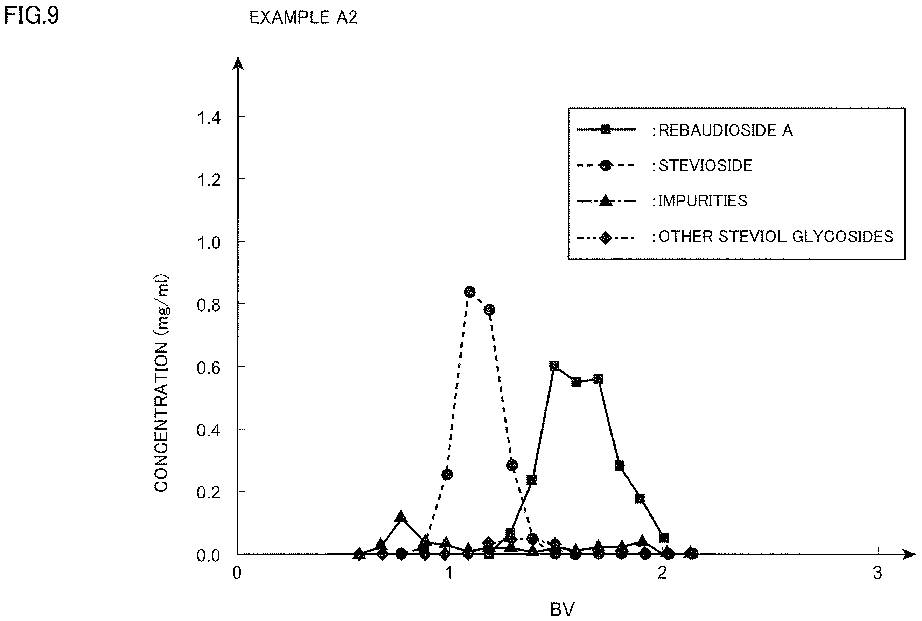

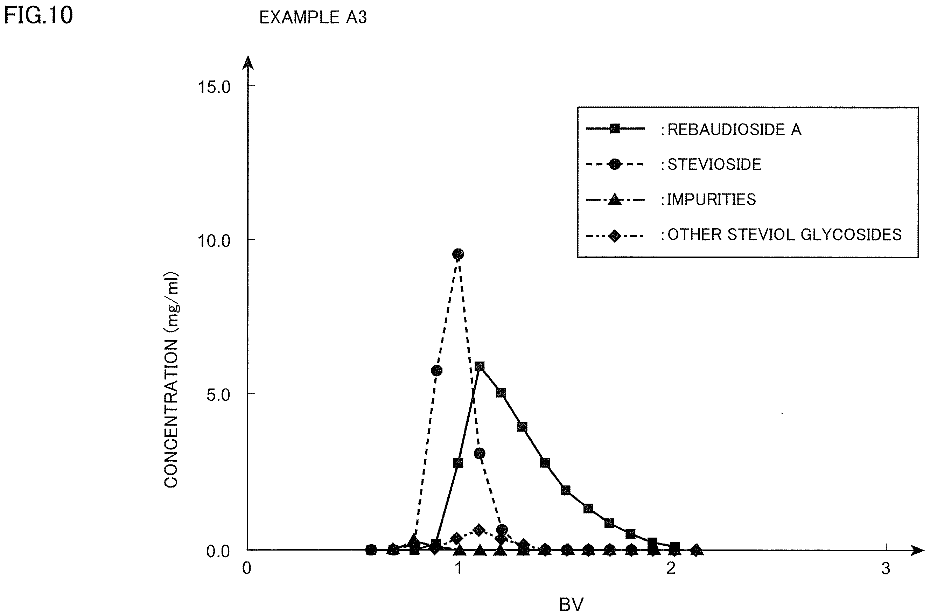

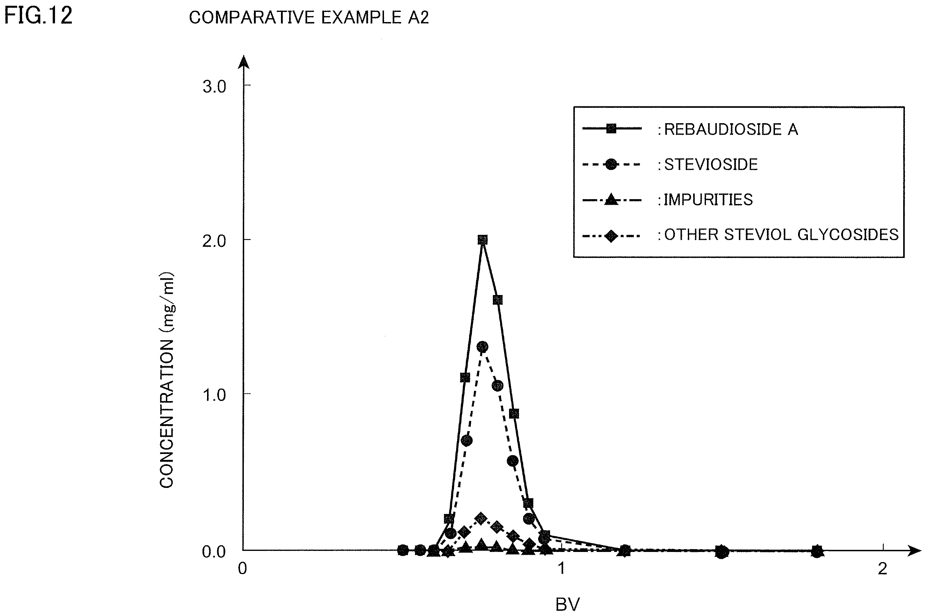

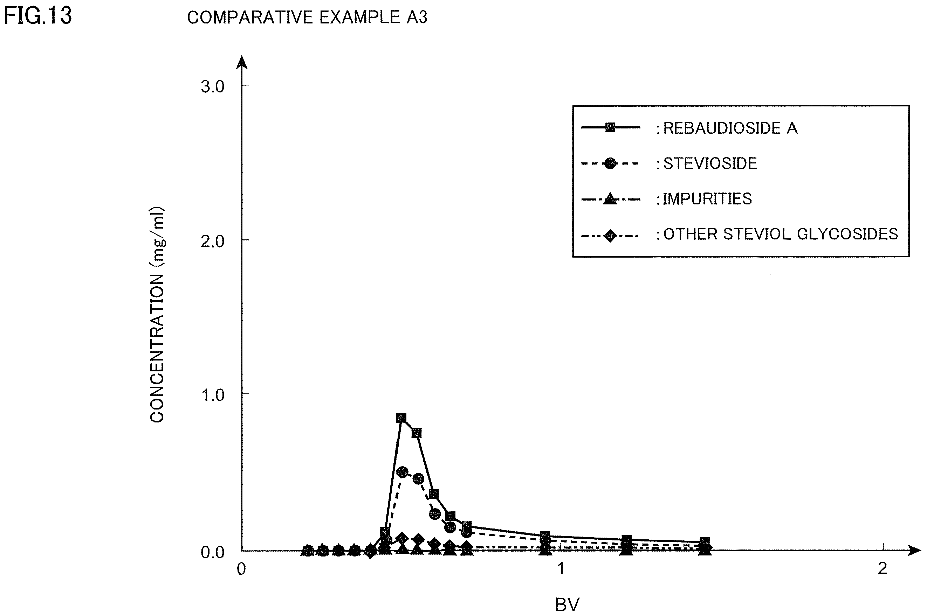

The results of Examples A1 to A3 and Comparative Examples A1 to A3 are respectively illustrated in Fig. 8 to Fig. 13.

Fig. 8 to Fig. 13 are diagrams illustrating the chromatograms of Examples A1 to A3 and Comparative Examples A1 to A3. Here, a horizontal axis represents a bed volume (BV), and a vertical axis represents the concentration of the component contained in the separated liquid.

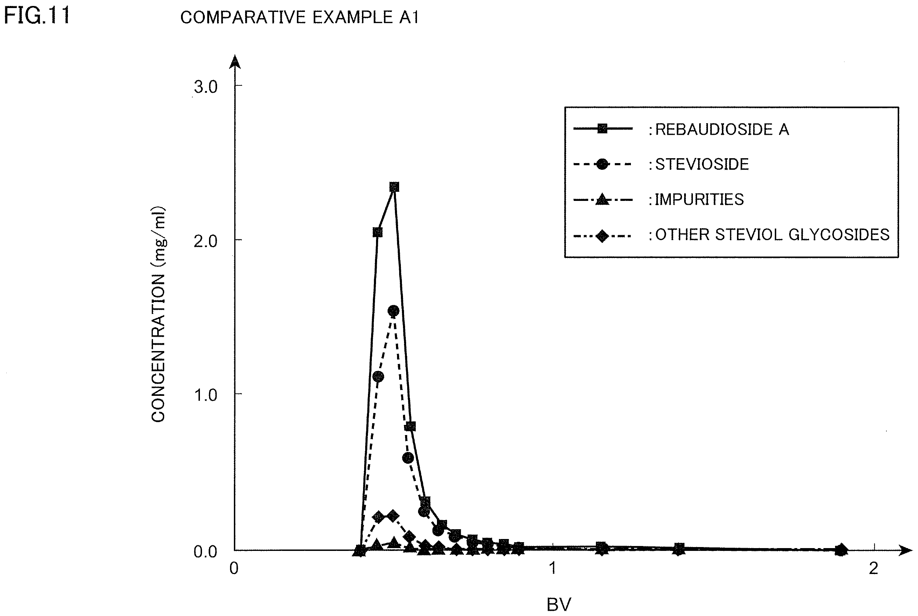

In the case of comparing Fig. 8 to Fig. 10 that are the results of Examples A1 to A3 with Fig. 11 to Fig. 13 that are the results of Comparative Examples A1 to A3, in Examples A1 to A3, it is found that the rebaudioside A and the stevioside are separated, whereas in Comparative Examples A1 to A3, the rebaudioside A and the stevioside are simultaneously eluted without being adsorbed in the ion exchange resin, and thus, are not capable of being separated. Note that, in Comparative Example A3, the peak of the rebaudioside A and the stevioside that are eluted is low, and the concentration thereof is lower than the others. According to a check, only approximately 60% of the rebaudioside A and the stevioside was eluted in the separated liquid, and the remaining 40% remained adsorbed in the ion exchange resin. Note that, in Comparative Example A4, both of the rebaudioside A and the stevioside remained adsorbed in the ion exchange resin without being eluted.

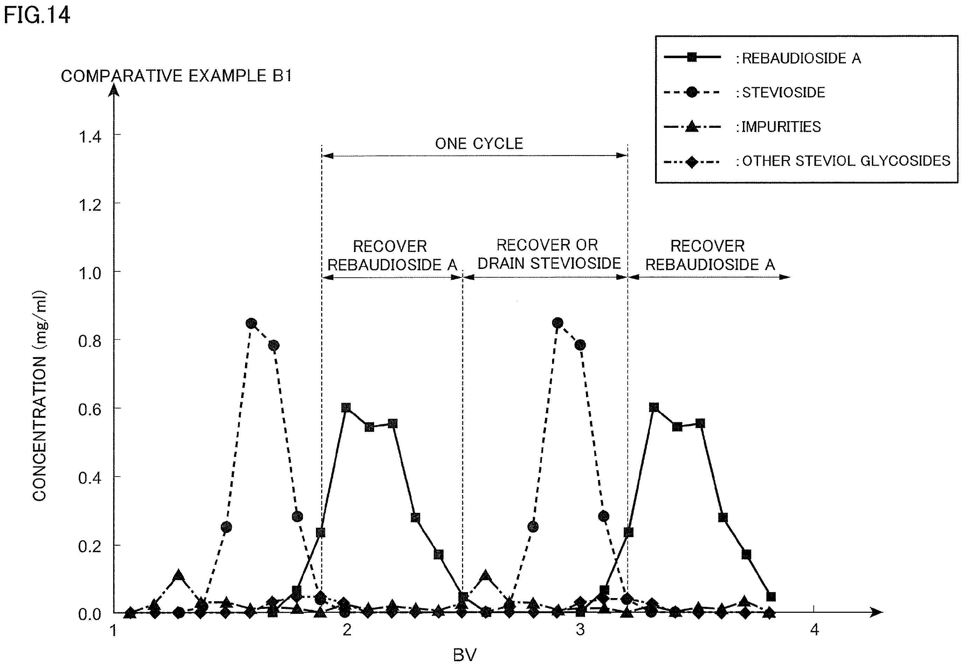

Fig. 14 is a diagram illustrating the chromatogram of Comparative Example B1. Here, a horizontal axis represents a bed volume (BV), and a vertical axis represents the concentration of the component contained in the separated liquid.

As illustrated, the rebaudioside A and the stevioside are alternately discharged, and the rebaudioside A and the stevioside are capable of being separately recovered.

In addition, the results of Example B1 and Comparative Example B1 are shown in Table 2 described below.

**[Table 2]**

| | | Example B 1 | Comparative Example B 1 |
|---|---|---|---|
| | | Simulated moving bed type | Single-pipe column continuous treatment |
| Operation condition | Concentration of raw material (mg/ml) | 10 | 10 |
| | Ratio | 51 : 44 | 51 : 44 |
| | SV (h⁻¹) | 1 | 1 |
| | Load (h⁻¹) | 0.058 | 0.038 |
| Result | Purity (%) | 95.87 | 95.32 |
| | Recovery rate (%) | 98.2 | 81.7 |
| Production | Yield (t/Year) | 10.00 | 10.00 |
| | Amount of separating agent (m³) | 10 | 14 |
| | Use amount of alcohol solvent (m^{3/}d) | 100 | 323 |
| | Use ratio of alcohol solvent | 1.0 | 3.2 |

In the case of using the simulated moving bed type chromatographic separation device 1 in Example B1, the purity of the rebaudioside A was 95.87%. Further, the recovery rate of the rebaudioside A was 98.2%. In contrast, in the case of using continuous pulse type chromatographic separation device in Comparative Example B2, the purity of the rebaudioside A was 95.32%, but the recovery rate was only 81.7%.

That is, in the case of comparing Example B1 with Comparative Example B1, the recovery rate was more excellent in Example B1 then in Comparative Example B1, at the same purity of the rebaudioside A.

When 10.00 t of the rebaudioside A was produced by operating the device for 180 days a year, the amount of separating agent to be used and a use amount of the alcohol solvent in the separating step 55 (refer to Fig. 1) were calculated. Accordingly, it was found that in Example B1, the amount of separating agent to be used was 10 m³, whereas in Comparative Example B1, 14 m³ of the separating agent was required. In addition, it was found that in Example B1, the amount of solvent to be used was 100 m³/d, whereas in Comparative Example B1, 323 m³/d of the solvent was required, and thus, approximately 3.2 times the amount of solvent in Example B1 was required as a use ratio of the alcohol solvent.

That is, in the case of comparing Example B1 with Comparative Example B1, it is possible to further reduce the amount of separating agent to be used or the use amount of the solvent in Example B1 than in Comparative Example B1. Accordingly, the cost for producing the rebaudioside A is less expensive in Example B1 than in Comparative Example B 1.

### EXPLANATIONS OF LETTERS OR NUMERALS

- 1, 100: CHROMATOGRAPHIC SEPARATION DEVICE
- 10 (11, 12, 13, 14): FILLING PORTION
- 20 (21, 22, 23, 24): SUPPLY PORTION
- 30 (31, 32, 33, 34): EXTRACTION PORTION
- 40: SWITCHING PORTION
- 54: ADSORBING STEP
- 55: SEPARATING STEP
- 59: DECOLORING STEP

## Claims

1. A method for separating steviol glycoside, comprising:
a separating step of performing a continuous liquid chromatography for continuously separating at least one type of steviol glycoside by allowing a liquid to be separated containing a plurality of types of steviol glycosides to pass through a separating agent in which polyethylene imine is immobilized to a carrier.

2. The method for separating steviol glycoside according to claim 1,
wherein the carrier is a macromolecular carrier.

3. The method for separating steviol glycoside according to claim 1,
wherein the liquid to be separated contains rebaudioside A as the steviol glycoside, and
the rebaudioside A is separated from the liquid to be separated.

4. The method for separating steviol glycoside according to claim 3,
wherein the liquid to be separated further contains stevioside as the steviol glycoside, and
each of the rebaudioside A and the stevioside is separated from the liquid to be separated.

5. The method for separating steviol glycoside according to claim 3,
wherein the liquid to be separated contains lower alcohol having carbon atoms of lower than or equal to 4 as a solvent.

6. The method for separating steviol glycoside according to claim 1,
wherein the continuous liquid chromatography is performed by using a simulated moving bed type device.

7. A method for producing rebaudioside A, comprising:
a separating step of performing a continuous liquid chromatography for continuously separating rebaudioside A by allowing a liquid to be separated containing a plurality of types of steviol glycosides containing the rebaudioside A to pass through a separating agent in which polyethylene imine is supported on a carrier.

8. A device for separating steviol glycoside, comprising:
a plurality of filling portions filled with a separating agent in which polyethylene imine for separating a plurality of types of steviol glycosides contained in a liquid to be separated by a chromatography is supported on a carrier;
a supply portion provided in each of the filling portions to supply each of the liquid to be separated and an eluent for extracting a separated liquid rich in at least one type of steviol glycoside in the liquid to be separated to the filling portion; and
an extraction portion provided in each of the filling portions to extract the separated liquid from the filling portion.
